(19) Europäisches Patentamt
European Patent Office
Office européen des brevets

(11) EP 1 748 805 B1

# (12) EUROPEAN PATENT SPECIFICATION

(45) Date of publication and mention of the grant of the patent:
**29.07.2009 Bulletin 2009/31**

(21) Application number: **05746512.2**

(22) Date of filing: **25.05.2005**

(51) Int Cl.:
***A61L 27/22*** *(2006.01)* ***A61L 27/34*** *(2006.01)*
***A61L 27/54*** *(2006.01)*

(86) International application number:
**PCT/GB2005/002081**

(87) International publication number:
**WO 2005/115492 (08.12.2005 Gazette 2005/49)**

## (54) ABSORBABLE BIOCOMPATIBLE COATINGS

RESORBIERBARE BIOKOMPATIBLE BESCHICHTUNGEN

REVETEMENTS BIOCOMPATIBLES ABSORBABLES

(84) Designated Contracting States:
**AT BE BG CH CY CZ DE DK EE ES FI FR GB GR HU IE IS IT LI LT LU MC NL PL PT RO SE SI SK TR**

(30) Priority: **25.05.2004 US 574250 P**

(43) Date of publication of application:
**07.02.2007 Bulletin 2007/06**

(73) Proprietor: **Biointeractions Ltd.**
**Reading, Berkshire RG6 6BZ (GB)**

(72) Inventors:
- **LUTHRA, Ajay Kumar**
  **Middlesex HA4 8QW (GB)**
- **SANDHU, Shivpal Singh**
  **Berkshire SL3 7PW (GB)**

(74) Representative: **Humphrey-Evans, Edward John et al**
**Humphrey-Evans**
**Intellectual Property Services Limited**
**1 Hawkes Close**
**Wokingham, Berkshire RG41 2SZ (GB)**

(56) References cited:
**WO-A-98/47948**
**WO-A-03/042277**
**WO-A-03/072156**
**US-A- 4 888 398**

- **DATABASE WPI Section Ch, Week 198933 Derwent Publications Ltd., London, GB; Class A96, AN 1989-236898 XP002349888 & JP 01 170464 A (LION CORP) 5 July 1989 (1989-07-05)**
- **TOSATTI S ET AL: "Peptide functionalized poly (l-lysine)-g-poly(ethylene glycol) on titanium: resistance to protein adsorption in full heparinized human blood plasma" BIOMATERIALS, ELSEVIER SCIENCE PUBLISHERS BV., BARKING, GB, vol. 24, no. 27, December 2003 (2003-12), pages 4949-4958, XP004464365 ISSN: 0142-9612**
- **YASUHARA NOSHO, TOSHINORI IKEHARA, YOSHIKO SASATANI, HIROAKI YAMAUCHI, SHINICHI HASHIMOTO: "Synthesis and antimicrobial activity of N-alpha-poly-acyl-N-epsilon-poly-L-lysine derivatives" CHEMISTRY EXPRESS, vol. 7, no. 10, 1992, pages 753-756, XP008054268 Japan**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 748 805 B1

## Description

### Field of the Invention

The field of the invention is generally related to bioabsorbable biocompatible coatings for delivery of therapeutic agents, in particular coatings made with polypeptides modified with hydrophobic side chains.

### Background

Some medical devices release a therapeutic agent after introduction of the device into a patient. For example, vascular stents may release therapeutic agents after deployment in the vasculature. The therapeutic agent may be deployed in a carrier material, also referred to as a vehicle, that is not absorbable and/or degradable.

### Summary of the Invention

Biocompatible carrier materials are described herein for enhanced delivery of therapeutic agents. The degradable material includes at least one amino acid that has been modified to include a hydrophobic side chain. The number and type of amino acids and hydrophobic side chains may be altered to adjust the properties of the material. Hydrophilic groups may also be introduced as a further means to adjust the material's property.

Some embodiments are directed to a coating and a method of making a coating, e.g., on a surface of a medical device, by forming (an optionally biodegradable) layer on at least a portion of the surface of the medical device, the layer comprising a copolymer and a therapeutic agent releasable into a patient after implantation of the device into the patient, wherein the copolymer comprises a first monomer unit and a second monomer unit, with the first monomer unit comprising an amino acid and the second monomer unit comprising an amino acid derivatized to have a hydrophobic hydrocarbon side chain that has a molecular weight from about 14 to about 5000.

Other embodiments are directed to a medical device with a coating operable to deliver a therapeutic agent. Conveniently, the medical device has a coating comprising a layer of the therapeutic agent and water soluble polyamino acids on at least a portion of a surface of the medical device; the polyamino acids having been crosslinked to each other whereby to stabilize the layer, wherein between 1% and 100% of the side chains of the polyamino acids are derivatized to have a hydrophobic hydrocarbon side chain that has a molecular weight from 14 to 5000.

### Discussion of the Prior Art

WO 03/042277 relates to a polymer comprising amino acid residues incorporated into a biodegradable polyester in which active drug molecules are attached to the amino acid side chains.

WO03/072156 relates to implantable bio-medical devices coated with a polyethylene glycol-grafted copolymer.

US 4,888,398 relates to esterified polypeptides, especially polyamino acids such as polyaspartate or polyglutamate. The polymer is biodegradable and may be used as a carrier for drug molecules.

WO 98/47948 relates to polymeric compositions for coating biological surfaces. These include polyethylene glycol/ polylysine (PEG/PLL) block or comb-type copolymers with high molecular weight PLL and PEG/PLL copolymers in which the PLL is a dendrimer which is attached to one end of the PEG.

JP 01 170464 relates to a polymer for coating an implant which comprises a polyamino acid, for example polylysine or polyarginine.

Tosatti et al, Biomaterials, (2003), 27, 4949-4958 relates to a graft copolymer poly(L-lysine)-*graft*-poly(ethylene glycol) and its RGD- and RGD-functionalized derivatives. When titanium surfaces were modified with these polymers, they showed no statistically significant protein adsorption during exposure to plasma for 30 min at 22°C or 37°C which suggests that the polymers are useful candidates for the surface modification of biomedical devices.

Yashura et al, Chemistry Express, (1992), 7, 753-756 relates to polyamino acid polymers which have antimicrobial activity.

### Brief Description of the Figures

Figure 1 depicts the cumulative release of a therapeutic agent from an 18 mm stent coated with a derivatized polyamino acid;

Figure 2 depicts the embodiment of Figure 1, with the release of the therapeutic agent being depicted on a per day basis;

Figure 3 depicts the cumulative release of a therapeutic agent, from 12 mm stents coated with three different polyamino acids being adjusted to have hydrophobic side chains of different lengths;

Figure 4 depicts the embodiment of Figure 3, with the release of the therapeutic agent being depicted on a per day basis.

Detailed Description of Preferred Embodiments

Materials with amino acids with side chains modified to have hydrophobic moieties may be used as coatings on medical devices to deliver drugs, and these materials can be referred to as carrier materials. In some embodiments, polyamino acids are decorated with hydrophobic groups to change the solubility of the polyamino acids in organic solvents. The degree of a derivatized polyamino acid's hydrophobicity may be controlled to influence compatibility with solvents and the rate of release of drugs associated with the polyamino acid. Further, hydrophilic groups may be introduced into the polyamino acids to further control solubility and release profiles. An advantage of this material is that its properties can be tailored to match the characteristics of the therapeutic agent that the material releases. For example, a polyamino acid may be decorated with hydrophobic groups as needed to solubilize the polyamino acid in a solvent that is desired for a particular therapeutic agent. Then the polyaminoacid and the agent may both be solubilized in the same solvent to form a composition that may be deposited as a layer on a surface of a medical device.

Medical devices, such as stents, catheters, guidewires, vascular grafts, wound closure devices and the like are continually being used in many clinical procedures. The performance of these devices can be enhanced by delivery of therapeutic, diagnostic, or other agents to site specific regions within the patient and it is desirable to provide the delivery from a degradable and biocompatible vehicle.

The biocompatible carrier materials may be degradable, and/or absorbable, and/or clearable. Degradable refers to a process of breaking down into smaller pieces. Hydrolytic degradation is a process of degradation that is facilitated by exposure to water. Bioactive degradation is a process of degradation that is mediated by a cell or cellular product, and includes the action of proteases, enzymes, and free radical attack by macrophages. Absorbable is a term used herein to indicate materials that break down into components that can be used, recycled, or completely destroyed by the body's biological processes. For example, amino acids, polypeptides, and fatty acids are believed to be absorbed after their introduction into the body, and may be directly integrated into metabolic processes or may be destroyed, e.g., in a lysosome. Clearable materials are materials that are cleared from the body, for example, via urine or sweat.

Currently, there is significant commercial interest of drug delivery from stents that utilize nonabsorbable and nondegradable vehicles where there is a possibility of long term toxic reaction between the host and the non-absorbable delivery vehicle. To address these issues, embodiments are described herein that include polypeptide derivatives that are biocompatible and bioabsorbable. Their preparation, characterization and method of use are also described.

Amino acid is a term used herein to refer not only to a free amino acid, but also to amino acids that have been reacted with each other, or with linking groups. When reacted to form a larger molecule, the amine terminal group and the carboxylic acid terminal group of the amino acid can be recognized within the larger structure such that the presence of the amino acid can be identified. An amino acid may be a natural or synthetic amino acid. Natural refers to an amino acid found in nature, while synthetic refers to an amino acid not found in nature. Amino acids include D or L forms, and those amino acids having a derivatized backbone. Certain embodiments are directed to derivatized amino acids, meaning a chemical structure that can be achieved by adding or substituting groups the an amino acid's N-terminus, C-terminus, side chain, or all three. In some cases, a molecule is referred to as being decorated, meaning that a group has been added to a molecule, usually by replacement of at least one other group.

An amino acid may be a moiety derived from a natural amino acid, e.g., alanine, arginine, asparagines, aspartic acid, cysteine, glutamine, glutamic acid, glycine, histidine, isoleucine, leucine, lysine, methionine, phenylalanine, proline, serine, threonine, tryptophan, tyrosine and valine. Natural amino acids have a general formula of $H_2N-CH[R]-C(=O)-OH$ (Formula 1), wherein R is an amino acid side chain, e.g., for lysine R is $-(CH_2)_4-NH_2$ (referred to as $R_1$) and in the case of glutamate, R is $-(CH_2)_2-C(=O)-OH$ (referred to as $R_2$). For the case of alanine, R is $-(CH_3)$ (referred to as $R_3$). Thus $-[HN-CH[R_1]-C(=O)]_n-$ is the formula for polylysine with n being the number of repeat units. As depicted, polylysine is a homo-amino acid sequence, meaning it has all the same type of amino acids. A heteroamino acid sequence comprises at least two types of amino acids, e.g., $-[HN-CH[R_1]-C(=O)]_n-[HN-CH[R_2]-C(=O)]_m-[HN-CH[R_3]-C(=O)]_q-$ (Formula 2), with n, m, and q designating a number of repeat units. The formulae for R for natural amino acids are well known. The C that is bonded to the R is referred to as the alpha carbon.

Polyamino acids may have a formula of $H_2N-\{CH[R]-L\}_n$ - COOH (Formula 3) wherein R is a side chain group and L is a linking group between the alpha carbons. The structure of L will depend on the groups on the alpha carbon of an amino acid that are reacted to form the linkages between the alpha carbons. In the case of a natural amino acids, a carboxyl and an amino group are reacted to form an amide bond so that L is C(=O)-NH. Other linking groups are

applicable for polypeptides having alternative backbones. Alternatively, a polyaminoacid may be a gamma polyaminoacid formed through amino acid gamma carbons, e.g., wherein the N-terminus of an amino acid is reacted with a carboxylic acid of a side chain of another amino acid to form a bond. A gamma polyamino acid may have some or all of the amino acids therein linked via the gamma carbons.

The N-terminus and C-terminus of a polypeptide (a term used interchangeably with polyamino acid) may be modified using chemistries known to persons of ordinary skill in these arts so that a polypeptide may be represented as Yn-{CH[R]-L}$_n$ - Yc, with Yn and Yc being independently chosen to be H, a halogen, a hydroxyl group, a thiol group, a carboxyl group, an amino group, an alkyl group, an alkoxy group, an alkenyl group, or an alkynyl group. Alternatively, Yn and/or Yc may be chosen to be X*, as set forth with reference to Formula 4, below.

An embodiment is a polypeptide that comprises a chemical having a formula of A-Z-T (Formula 4), wherein A is a natural or synthetic amino acid derivatized with Z and T; Z is a bond, or a linking group that links the indicated groups, A and T, by covalent bond(s) and may include an aliphatic group and at least one heteroatom such as P, O, S, and N, the aliphatic group may be, e.g., an alkane, alkene, alkyne, or combinations thereof, the linking group Z may include, e.g., between about 1 and about 100 atoms; and T has a formula of H or $-(CH_2)_n-X^*$ group, wherein X* is a H, a halogen, a hydroxyl group, a thiol group, a carboxyl group, an amino group, an alkyl group, an alkoxy group, an alkenyl group, or an alkynyl group, and $-(CH_2)_n$ is a group where n is an integer between 1 and about 100, inclusive, and one or more of the methylene groups is optionally replaced by O, S, N, C, C=O, an $NR_a$ group, a $CR_b$ group, a $CR_cR_d$ group, or a $SiR_eR_f$ where $R_a$, $R_b$, $R_c$, $R_d$, $R_e$, and $R_f$ are, each independently, a bond, a pi bond, H, a hydroxyl group, a thiol group, a carboxyl group, a carbamate, an oxocarbon group, an amino group, an amido group, an amide group, a phosphate group, a sulfonate group, an alkyl group, an alkoxy group, an alkenyl group, an alkynyl group, a siloxane or a functional group.

In one embodiment, T comprises a hydrophobic aliphatic group with n being between about 2 and about 100, and may include an aliphatic group and at least one heteroatom such as P, O, S, and N. The aliphatic group may be, e.g., an alkane, alkene, alkyne, or combinations thereof. The aliphatic chain may, furthermore, have at least one, or between about 1 and about 50 hydrophobic aliphatic groups; persons of ordinary skill in these arts will immediately appreciate that every value and range within the explicitly articulated range of 1-50 is contemplated. It is noted that Z and T may therefore be chosen to be any side chain of an amino acid. It is further noted that Z may be chosen to be a bond and T may be chosen to be H so that a $CH_2$ group is formed on a backbone.

An embodiment is a material that comprises a chemical having a formula of

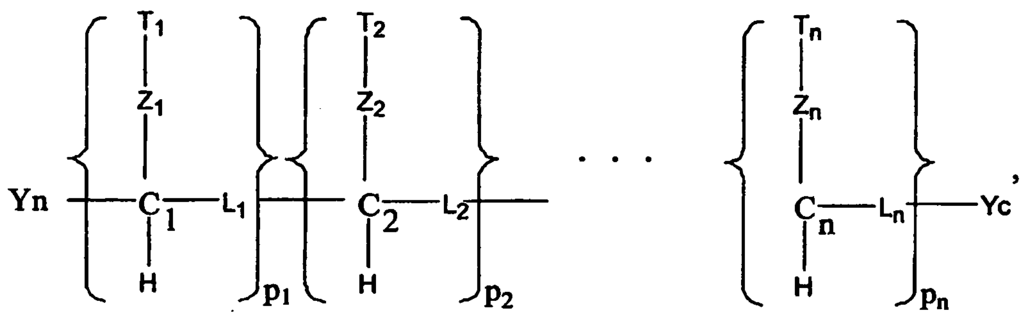

Formula 5,

or a material that comprises a chemical comprising a formula of

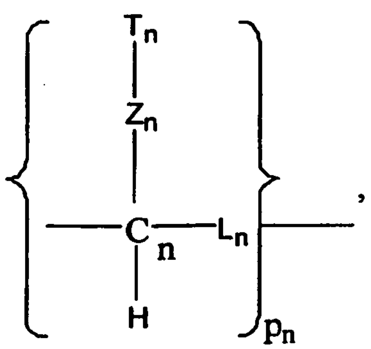

Formula 6,

wherein

p is an integer that indicates a monomer unit of the polymer

n is an integer between about 1 and about 500,000;

$T_1 . . . T_n$ are independently chosen to have a formula of H or-$(CH_2)_n$-X* group, wherein X* is a H, a halogen, a hydroxyl group, a thiol group, a carboxyl group, an amino group, an alkyl group, an alkoxy group, an alkenyl group, an alkynyl group, and -$(CH_2)_n$ is a group where n is an integer between 1 and about 100, inclusive, and one or more of the methylene groups is optionally replaced by O, S, N, C, C=O, an $NR_a$ group, a $CR_b$ group, a $CR_cR_d$ group, or a $SiR_eR_f$ where $R_a$, $R_b$, $R_e$, $R_d$, $R_e$, and $R_f$ are, each independently, a bond, a pi bond, H, a hydroxyl group, a thiol group, a carboxyl group, a carbamate, an oxocarbon group, an amino group, an amido group, an amide group, a phosphate group, a sulfonate group, an alkyl group, an alkoxy group, an alkenyl group, an alkynyl group a siloxane or a functional group, $L_1 . . . L_n$ are independently chosen to be a bond, or a linking group that links the indicated carbons by covalent bond(s) and may include an aliphatic group and at least one heteroatom such as P, O, S, and N, the aliphatic group may be, e.g., an alkane, alkene, alkyne, or combinations thereof, L may include, e.g., between about 1 and about 100 atoms; $Z_1 . . . Z_n$ are independently chosen to be a bond, or a linking group that links the indicated C to T, by covalent bond(s) and may include an aliphatic group and at least one heteroatom such as P, O, S, and N, the aliphatic group may be, e.g., an alkane, alkene, alkyne, or combinations thereof, the linking group Z may include, e.g., between about 1 and about 100 atoms; and Yc and Yn are each independently chosen to be H, a halogen, a hydroxyl group, a thiol group, a carboxyl group, an amino group, an alkyl group, an alkoxy group, an alkenyl group, an alkynyl group a siloxane or a functional group. Alternatively, Yn and/or X* may be defined as X*, as set forth with reference to Formula 4, above.

By way of example, Formula 6 with n=3 would have a formula of:

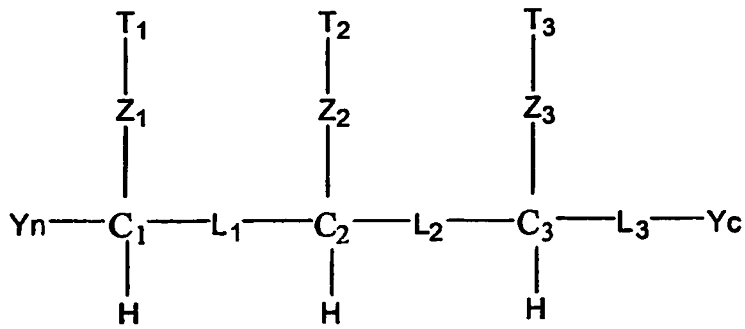

**(Formula 6a),**

wherein $T_1$, T2, and T3, are each chosen independently, as may be $Z_1$, $Z_2$, and $Z_3$.

In certain embodiments, referring to Formulas 5-8, Tn, or Tn and Zn together, with Tn having a formula of H or -$(CH_2)_n$-X* group, comprises a hydrophobic aliphatic group with n being between about 1 and about 100, and may include an aliphatic group and at least one heteroatom such as P, O, S, and N. The aliphatic group may be, e.g., an alkane, alkene, alkyne, or combinations thereof. Persons of ordinary skill in these arts will immediately appreciate that every value and range within the explicitly articulated range is contemplated, not only for the range immediately preceding this statement, but for other ranges set forth herein.

In certain embodiments Tn comprises between about 4 and about 100 $(CH_2)$ with between about 1 and about 50 of the $(CH_2)$ being substituted with at least group having at least one heteroatom such as P, O, S, and N.

In certain embodiments, a compound of Formulas 5-8, Tn comprises a functional group, FG, for forming a covalent bond with another functional group, e.g.,: as shown in Formula 7:

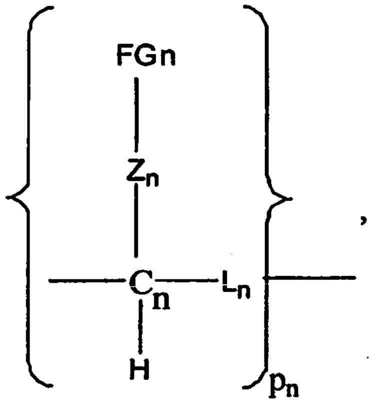

, Formula 7,

wherein FGn denotes an optional functional group $FG_1$. . . FGn. Examples of FG are: polymerizable groups (e,.g., vinyl groups, groups polymerizable by free radical, addition, condensation polymerization) a carboxyl, amino, hydroxyl or acid chloride, anhydride, isocyanate, thiocyanate, azides, aldehyde, ketone, thiol, allyl, acrylate, methacrylate, epoxide, aziridines, acetals, ketals, alkynes, acyl halides, alky halides, hydroxy aldehydes and ketones, allenes, amides, bisamides, and esters, amino carbonyl compounds, mercaptans, amino mercaptans, anhydrides, azines, azo compounds, azoxy compounds, boranes, carbamates, carbodimides, carbonates, diazo compounds, isothionates, hydroxamic acid, hydroxy acids, hydroxy amines and amides, hydroxylamine, imines, lactams, nitriles, sulphonamides, sulphones, sulphonic acids and thiocyanates.

The Formulas 5-8 may thus be used to describe compounds that are polymers, block polymers, or random polymers. By way of an example a polypeptide backbone can consist of lysine where $R_1$ is-$(CH_2)_4$-$NH_2$ and as such the amine is able to chemically react with an acid chloride, an acid anhydride, isocyanate, and with other functional groups. A polypeptide backbone as found in nature has an amide linking group, as shown in the embodiment of Formula 8:

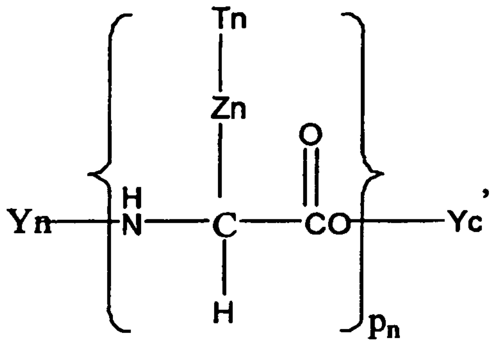

, Formula 8,

wherein Yn, and Yc are defined as set forth in reference to Formulas 5 and 6, above. In some embodiments Tn is defined as set forth in reference to Formulas 5 and 6; alternatively Tn may be chosen only to comprise H or a linear or branched aliphatic group, e.g., $C_1$-$C_{50}$, or $C_4$ - $C_{25}$. Alternatively, substitution of heteroatoms into the aliphatic may also be employed, e.g., O, S, P, and N. In some embodiments Zn is defined as set forth in reference to Formulas 5 and 6; alternatively Zn may be chosen as a bond or a linker that comprises between 2 and 5, 10, 15, or 20 atoms, and forms a covalent bond between Tn and C. Alternatively, Zn may be chosen to be a bond or an amino acid side chain that is derivatized to form a bond between the side chain and Tn.

Polypeptides, polypeptide backbones, or molecules comprising polypeptides may be homoaminoacid or heteroaminoacid sequences. For example, a polypeptide may be a block copolymer, an alternating copolymer or a random polymer. For a block copolymer, each block can have a set of the same amino acid types, e.g., KKKAAA, KKKAAAKKK, or KKKKKAAAKKAAAKKKKAAAA, wherein K and A are lysine and alanine, respectively. For alternating copolymers, the polymer can have a specific ordered structure, such as KAKAKAKAKAK, KKAKKAKKAKKAKKA, or KKAAKKAAKKAAKKAA. The block copolymers and alternating copolymer can have more than two types of amino acids as desired. Or a polypeptide may be random, e.g., AKRCKKRACKRAAK, wherein A, K, C, and R are amino acids. Or a backbone may comprise linking groups between amino acids, e.g., AKR-Z-AKR-Z-KKA-Z-C-Z-AA-wherein A, K, R, and C are amino acids and Z is a linking group, e.g., as defined herein with reference to Formulas 5 and 6. The side chains of the amino acids maybe protected, unprotected, or derivatized, e.g., with aliphatic chains, e.g., $C_2$ to $C_{50}$. An

embodiment is a polypeptide having a formula of:

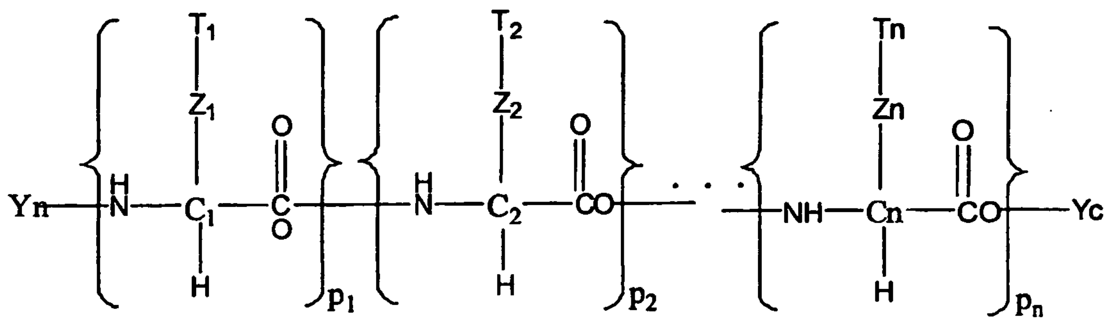

(Formula 9),

wherein $p_1$, $p_2$, . . . $p_n$ are units in a polypeptide sequence, and may be an amino acid with/without, a derivitized side chain. Yn, Tn, Zn and Yc may be defined as defined for other embodiments herein, e.g., Formulas 5-8. Thus the embodiment of Formula 9 may have a backbone that is a heteroaminoacid, a homoamino acid, and may have a block or random sequence.

In some embodiments, side chains of amino acids in polypeptides may be derivatized with hydrocarbon chains, for example, saturated or mono-unsaturated or polyunsaturated. Such long chain hydrocarbons are, but not limited to, lauric acid, myristic acid, palmitic acid, stearic acid, oleic, acid, erucic acid, linoleic acid, (alpha)-linolenic acid, arachidonic acid, eicosapentaenoic acid, docosahexaenoic acid, 1-eicosanol, undecylamine, dodecylamine, dodecyl isocyanate, dodecenyl succinic anhydride, dodecanol, dodecanal, dodecanthiol, dodecanolactone, 2-dodecenedioic acid, cis-5-dodecenoic acid, cis-7-dodecen-1-ol, hexadecylamine, cis-9-hexadecenal, cis-11-hexadecen-1-ol, hexadecenyl isocyanate, hexadecanethiol, hexadecanol, hexadecanesulphonyl chloride, hexadecanoic anhydride, hexadecyl isocyanate, heptadecanol, 1-nonadecanol, 1-octadecanol, 1-octadecane thiol, octadecyamine, octadecylamide, octadecyl isocyanate, pentadecylamine, pentadecane thiol, palmitoleic acid, tridecylamine, tridecanal, tridecanol, tetradecylamine, tetradecyl aldehyde, cis-11-tetradecen-1-ol, tetradecyl isocyanate. Thus, for example, Tn may be selected as a hydrocarbon chain, e.g., from $C_1$ to $C_{50}$. And, for example, such polypeptides may be between, e.g., about 4 and about 100,000 or between about 6 and about 10,000 amino acids in length, or with a molecular weight of amino acids between about 1000 - 4 million.

The term polypeptide is used herein to include embodiments that comprise a natural or synthetic amino acid that has been decorated with an alternate chemical moiety. Preparation of polypeptides and derivatized polypeptides can be performed by persons of ordinary skill in these arts after reading this disclosure. Polypeptide chemistry references include, e.g., M. Bodanszky, "Peptide Chemistry", Springer-Verlag, 1988; Norbert Sewald, Peptides: Chemistry and Biology, Wiley, John & Sons, Incorporated, August 2002; Gregory A. Grant, Synthetic Peptides: A User's Guide, Oxford University Press, March 2002; sources that provide additional details for reaction schemes for derivatizing peptides: T. W. Greene, Protective Groups in Organic Synthesis, 3rd Edition; Wiley: New York, 1999; R. C. Larock, Comprehensive Organic Transformations: A Guide to Functional Group Preparations, New York, 1989; B. M. Trost, I. Fleming (eds.-in-chief), Comprehensive Organic Synthesis: Selectivity, Strategy & Efficiency in Modem Organic Chemistry: Cumulative Indexes, Volume 9.

*Substitution and substituents*

Substitution is liberally allowed on chemical groups, and on the atoms that occupy a position in a Formula depicted herein, for various physical effects on the properties of the compounds, such as mobility, sensitivity, solubility, compatibility, stability, and the like, as would be known to a person of ordinary skill in these arts after reading this disclosure. In the description of chemical substituents, there are certain practices common to the art that are reflected in the use of language. The term group indicates that the generically recited chemical entity (e.g., alkyl group, alkenyl group, aromatic group, epoxy group, arylamine group, aromatic heterocyclic group, aryl group, alicyclic group, aliphatic group, heterocyclic non-aromatic group etc.) may have any substituent thereon which is consistent with the bond structure of that group. For example, where the term 'alkyl group' is used, that term would not only include unsubstituted linear, branched and cyclic alkyls, such as methyl, ethyl, isopropyl, tert-butyl, cyclohexyl, dodecyl and the like, but also substituents having heteroatom such as 3-ethoxylpropyl, 4-(N-ethylamino)butyl, 3-hydroxypentyl, 2-thiolhexyl, 1,2,3-tribromoopropyl, and the like. However, as is consistent with such nomenclature, no substitution would be included within the term

that would alter the fundamental bond structure of the underlying group. For example, where a phenyl group is recited, substitution such as 1-aminophenyl, 2,4-dihydroxyphenyl, 1,3,5-trithiophenyl, 1,3,5-trimethoxyphenyl and the like would be acceptable within the terminology, while substitution of 1,1,2,2,3,3-hexamethylphenyl would not be acceptable as that substitution would require the ring bond structure of the phenyl group to be altered to a non-aromatic form because of the substitution.

The term alkyl, unless otherwise specified, refers to a saturated straight, branched, or cyclic hydrocarbon, and specifically includes, e.g., methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl. The alkyl group can be optionally substituted with any appropriate group, including but not limited to one or more groups selected from halo, hydroxyl, amino, alkylamino, arylarmino, alkoxy, aryloxy, nitro, cyano, sulfonic acid, sulfate, phosphonic acid, phosphate, or phosphonate, either unprotected, or protected as necessary, as known to those skilled in the art. The term alkenyl, unless otherwise specified, is a straight, branched, or cyclic (in the case of $C_{5-6}$) hydrocarbon with at least one carbon-carbon double bond, and may be substituted as described above. The term alkynyl, unless otherwise specified, is a hydrocarbon, straight or branched, with at least one triple one carbon-carbon bond, and may be substituted as described above. In some embodiments, it is useful to limit the size of these substituents to, e.g., less than about 150, less than about 100, less than about 50, or less than about 20 atoms.

Other suitable substituent groups include these and other N-containing compounds e.g, amines, amides, amidium ions, amine imides, amine oxides, aminium ions, aminonitrenes, nitrenes, aminoxides, nitriles, and nitrile imides. Other suitable substituent groups include these and other S-containing compounds, e.g., sulfonic acid, sulfate, sulfonates, sulfamic acids, sulfanes, sulfatides, sulfenamides, sulfenes, sulfonic acids, sulfenium ions, sulfenyl groups, sulfenylium ions, sulfenyl nitrenes, sulfenyl radicals, sulfides, sulfilimines, sulfimides, sulfimines, sulfinamides, sulfinamidines, sulfines, sulfinic acids, sulfinic anhydrides, sulfinimines, sulfinylamines, sulfolipids, sulfonamides, sulfonamidines, sulfonediimines, sulfones, sulfonic acids, sulfonic anhydrides, sulfonamides, sulfonium compounds, sulfonphthaleins, sulfonylamines, sulfoxides, sulfoximides, sulfoximines, sulfur diimides, thiols, thioacetals, thioaldehydes, thioaldehyde S-oxides, thioanhydrides, thiocarboxylic acids, thiocyanates, thioethers, thiohemiacetals, thioketones, thioketone S-oxides, thiolates, and thionylamines. Other suitable substituent groups include these and other O-containing compounds, e.g., having the form ROH(alcohol), RCOOH (carboxylic acids), RCHO (aldehydes), RR'C=O (ketones), ROR' (ethers), and RCOOR' (esters), with the R denoting a bond or atomic element. Other suitable substituent groups include these and other P-containing compounds, e.g., phosphanes, phosphanylidenes, phosphatidic acids, phosphazenes, phosphine oxides, phosphines, phosphinic acids, phosphinidenes, phosphinous acids, phosphoglycerides, phospholipids, phosphonic acids, phosphonitriles, phosphonium compounds, phosphonium ylides, phosphono, phosphonous acids, phosphoramides, and phosphoranes. Carbon is useful for making substituents and the number of carbons in a heteroatomic structure may be, e.g., between 1 and n-1 when between 2 and n atoms are used to form a substituent with, e.g., O, P, S, or N. In some embodiments, it is useful to limit the size of these substituents to, e.g., less than about 150, less than about 100, less than about 50, or less than about 20 atoms.

The formulas set forth herein describe a variety of groups. All of these various groups may be optionally derivitized with substituent groups, as already described. Suitable substituent groups that may be present on such a "substituted" group include e.g., halogens such as fluoro, chloro, bromo and iodo; cyano; H, hydroxyl group; ester group; ether group; a carbamate, an oxo acid group, an oxocarbon group, an oxo carboxylic acid group, an oxo group, a ketone group; nitro; azido; sulfhydryl; alkanoyl e.g., $C_{1-6}$ alkanoyl group such as acetyl and the like; carboxamido; alkyl groups, alkenyl and alkynyl groups including groups having one or more unsaturated linkages; alkoxy groups having one or more oxygen linkages; aryloxy such as phenoxy; alkylthio groups; alkylsulfinyl groups; alkylsulfonyl groups; aminoalkyl groups such as groups having one or more N atoms; carbocyclic aryl; aryloxy such as phenoxy; aralkyl having 1 to 3 separate or fused rings; aralkoxy having 1 to 3 separate or fused rings; or a heteroaromatic, heterocyclic, or heteroalicyclic group having 1 to 4 separate or fused rings e.g., with one or more N, O or S atoms, e.g., coumarinyl, quinolinyl, pyridyl, pyrazinyl, pyrimidyl, furyl, pyrrolyl, thienyl, thiazolyl, oxazolyl, imidazolyl, indolyl, benzofuranyl, benzothiazolyl, tetrahydrofuranyl, tetrahydropyranyl, piperidinyl, morpholino and pyrrolidinyl. Other substituents may include groups that include O, S, Se, N, P, Si, C and have between 2 and about 150 atoms. In some embodiments, it is useful to limit the size of any substituent to, e.g., less than about 150, less than about 100, less than about 50, or less than about 20 atoms. Functional groups may also be substituted freely onto groups described herein; examples of such groups are described as FG in reference to Formula 7.

*Hydrophobic and hydrophilic groups for selected properties*

In some embodiments, the carrier material comprises a molecule having at least a portion that comprises a polypeptide backbone comprising homoamino acid sequences and/or heteroamino acid sequences, whereby the hydrophilic and hydrophobic balance is adjusted by chemical linkage to at least one side chain of the amino acids. Hydrophobic groups may be added to increase hydrophobicity and hydrophilic groups may be added to increase hydrophilicity.

Examples of hydrophobic groups are alkyls, alkenes, alkynes, aromatic groups (e.g., benzenes), many ringed structures (e.g., cyclohexane), and phospholipids.

Examples of hydrophilic groups are polyethylene oxides, sugar residues, polysaccharides, polyvinyl alcohols, polyethyleneimines, polyacrylic acids, and zwitterionic groups, e.g., a head group of a zwitterionic phospholipid.

Alternatively, the Tg of the polymer may be selected by addition of hydrophobic or hydrophilic groups; in such a case, a polymer is selected and hydrophobic or hydrophilic groups are added as needed to achieve a selected Tg. Alternatively, the addition of hydrophobic or hydrophilic groups may be adjusted to achieve a selected rate of release of a selected therapeutic agent; in such a case, for example as shown in Figures 3-4 , the release rate of the agent from layers of polypeptides with varying derivitization of their side chains is measured. The desired release profile is achieved by adjusting the number of derivatized side chains and the length of the hydrophobic or hydrophilic moieties on the side chains.

An unexpected and surprising result was that the relatively more hydrophobic materials released therapeutic agents more quickly than materials with a lower hydrophobic-to-hydrophilic ratio. This trend is evident in, for example, Figure 4, wherein paclitaxel was released most quickly with the relatively most hydrophobic material. Without being bound to a particular theory, the domains formed by the relatively longer hydrophobic portions of the polyaminoacid may have been more conducive to migration of the agent through the material. This acceleration of release is surprising and unexpected because relatively small changes in side chain length caused a disproportionate rate of increase in release. Further, this result is surprising because the rate of release would normally be expected to be highly related to the water solubility of the carrier material, with more soluble materials being more rapidly hydrated to accelerate release of agents therein. Accordingly, some embodiments are directed to polymers as described herein that have hydrophobic side chains of at least about 50-500 molecular weight.

In some embodiments the polypeptides, which are also referred to herein as polyaminoacids, have a molecular weight between about 1,000 and about 4,000,000. Alternatively, the molecular weights may be between about 20,000 and about 250,000. The molecular weight has an influence on coatings made with these materials. The side chains of a polypeptide may be partially derivatized or fully derivatized with respect to the total number of side chains, e.g., between about 1% and about 100%, or between about 3% and about 95% of the total number of side chains are derivatized. Persons of ordinary skill in these arts will immediately understand that all ranges and values within these explicitly stated molecular weight and derivitization ranges are contemplated.

An example is polylysine, which is chemically modified with a long chain hydrocarbon such as a stearoyl group: $---[HN-CH[R_1]-C(=O)-O]_n-[HN-CH[{}^*R_1]-C(=O)-O]_{(n-q)}---$, (Formula 10), wherein $^*R_1$ is $-(CH_2)_4-NH-C(=O)-[CH_2]_{16}-CH_3$ and where (n-q) is the degree of chemical modification. Alternatively, polylysine may be modified by attaching a hexane to its side chains, e.g., with an N-hydroxysuccinimide ester as shown in Example 1. The polylysine was soluble in aqueous solvents before modification, but, after being decorated with the hexanes, was insoluble in water. The derivatized polylysine was soluble in alcohols and dimethyl sulfoxide (DMSO) and dimethylformamide (DMF). About 60% of the side chains of the polylysine were modified with the hydrophobic groups and subsequent processing was used to modify about 94% of all of the side chains.

Various polyamino acids with various molecular weights and monomer units may be derivatized, e.g., polylysine (Example 1), polyglutamate (Example 4), polyaspartate (Example 5), and polyaminoacids having other amino acids. The degree of substitution can be controlled by altering the reactions, e.g., as in Example 1(ii) and 1(iii), so that substitution between about 1% and about 100% of the available side chains can be achieved. The length of hydrophobic and hydrophilic groups can be varied, e.g., as in Examples 1-8, so that, e.g., side chains comprising polyethylene glycol or $C_2$-$C_{50}$ may be achieved. Copolymers having different amino acids may be synthesized, e.g., as in Example 7, wherein aspartate and glutamates were copolymerized and their side chains were derivatized.

Further, derivatized portions of a polymer may be further derivatized to make them more hydrophobic or more hydrophilic. For instance, in Example 8, ethyl groups of derivatized polyaminoacid were converted to butyl groups using a transesterification process. And, for instance in Example 9, n-alkyl groups were transesterified to have glutamic acid side chains.

The higher the degree of chemical modification with hydrophobic moieties results in a shift of hydrophilic and hydrophobic balance to be more hydrophobic. The extent of chemical modification influences the bioabsorbable rate and hence the rate of delivery of the therapeutic agent or diagnostic agent.

By way of another example the polypeptide back bone is composed of glutamate and alanine and the glutamate units are chemically modified with a carbonyl diimadazole activated hydroxy long chain hydrocarbon:

$$-[HN-CH[{}^*R_2]-C(=-O)-O]_{(m-p)}-[HN-CH[R_3]-C(=O)-O]_q- \qquad \text{(Formula 11)},$$

wherein

$^*R_2$ is $-(CH_2)_2-C(=O)-O-[CH_2]_{16}-CH_3$ and where (m-p) is the degree of chemical modification and where $R_3$ is $-(CH_3)$.

Similarly, higher the degree of chemical modification results in a shift of hydrophilic and hydrophobic balance

to be more hydrophobic. The extent of chemical modification influences the bioabsorbable rate and hence the rate of delivery of the therapeutic agent or diagnostic agent.

By way of reference, BIORELEASE shall refer to a polypeptide backbone comprising of homoamino acids and or heteroamino acids sequences, whereby by chemical linkage to the polypeptide backbone it is possible to adjust the hydrophilic and hydrophobic balance. And, by way of reference, BIORELEASE-Lys30-Lauric50 shall refer to homopolypeptide backbone comprising lysine sequences, 30 shall refer to molecular weight range of polylysine, the backbone is chemically modified to incorporate lauric acid and 50 shall refer to the degree of incorporation of laurate group, this can be achieved by reaction of the corresponding acid chloride or other suitable functional group with the amine on the polypeptide backbone.

In another embodiment, drug loading into BIORELEASE is achieved by taking advantage the solubility of the drug and BIORELEASE in a suitable co-solvent such as iso-propylalcohol (IPA) / tetrahydrofuran (THF). Such an example is BIORELEASE-Lys30-Lauric50 and paclitaxel, which are found to be soluble in IPA / THF. Medical devices, such as stents, can be coated by dip-coating or spraying techniques. The release profile and the degradation rates may be measured in the laboratory.

In another embodiment where BIORELEASE-Lys1.5-Lauric50 is made to chemically couple with BIORELEASE-Glu1.5-Dodecan50 to give a final BIORELEASE composition of BIORELEASE (Lys15-Lauric50)-(Glu15-Dodecan50). BIORELEASE-Glu1.5-Lauric50 is prepared by the reaction of the acid group on the polypeptide backbone with carbonyl diimadazole activated 1-dodecanol. Similarly, drug loading into BIORELEASE is achieved by taking advantage the solubility of the drug and BIORELEASE in a suitable co-solvent such as IPA / THF. Such an example is BIORELEASE (Lys15-Lauric50)-(Glu15-Dodecan50) and paclitaxel, which are found to be soluble in IPA / THF. Medical devices, such as stents, can be coated by dip-coating or spraying techniques. The release profile and the degradation rates may be measured in the laboratory.

In another embodiment, individual amino acids are chemically modified by long chain hydrocarbons and these modified individual polypeptides are chemically linked to form BIORELEASE.

By way of an example the amino acid lysine is chemically modified with a long chain hydrocarbon such as stearoyl chloride to give (Lys-Stearic). The amino acid glutamate is chemically modified with carbonyl diimadazole activated 1-dodecanol to give (Glu-Dodecan) and this is followed by chemical coupling to yield the BIORELEASE: [(Lys-Stearic)$_n$-(Glu-Dodecan)$_m$]$_y$ (Formula 12), wherein n, m and y are the number of repeat units.

By way of reference this type of BIORELEASE shall be referred to as BIORELEASE-[(LS)$_n$-(GD)$_m$]$_y$.

Hydrophobic and/or hydrophilic groups and/or therapeutic agents may be attached to an amino acid side chain in a variety of ways. For example, these may be attached via an amide, ester, carbonate, carbamate, oxime ester, acetal, ketal, urethane, ureas, enol ester, oxazolindies, anhydride, or oxime ester. As an example, a carbamate linkage may be used as follows:

<u>Carbamate</u>

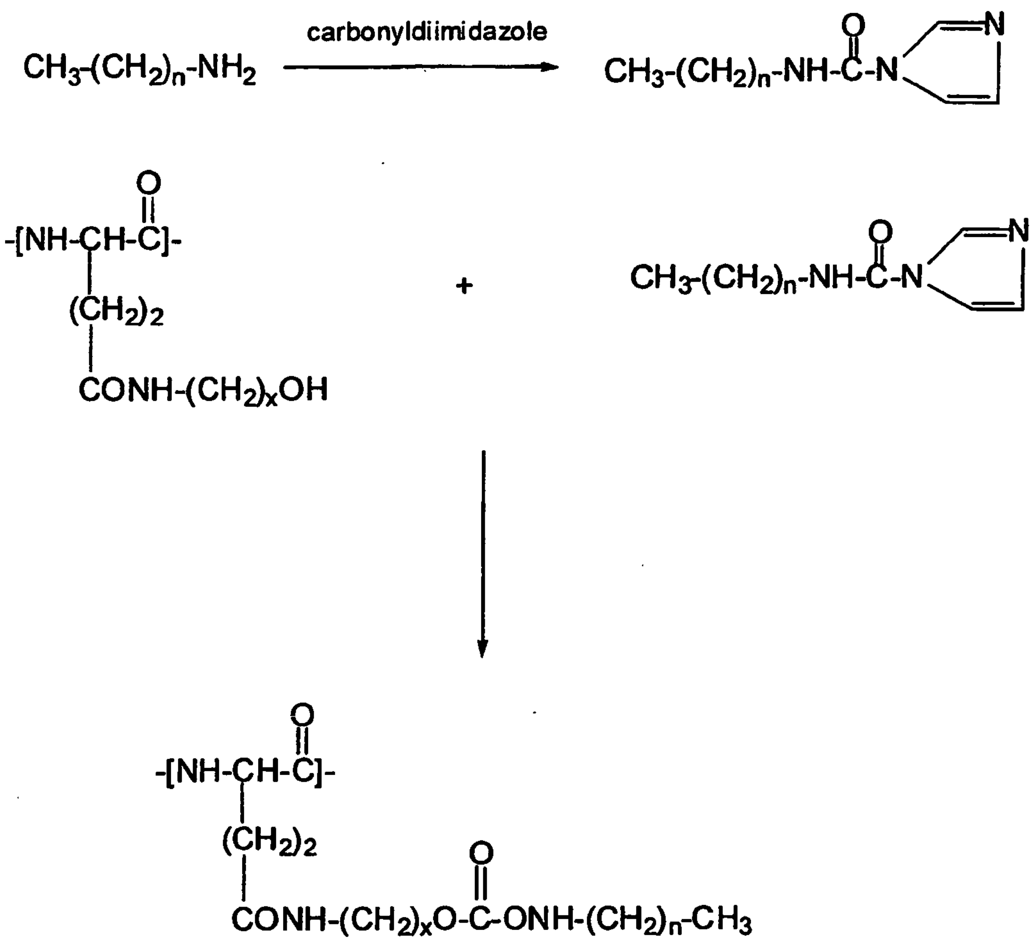


Reaction Scheme 1

Or a carbonate linkage may be used:

Carbonate Linkage

1.

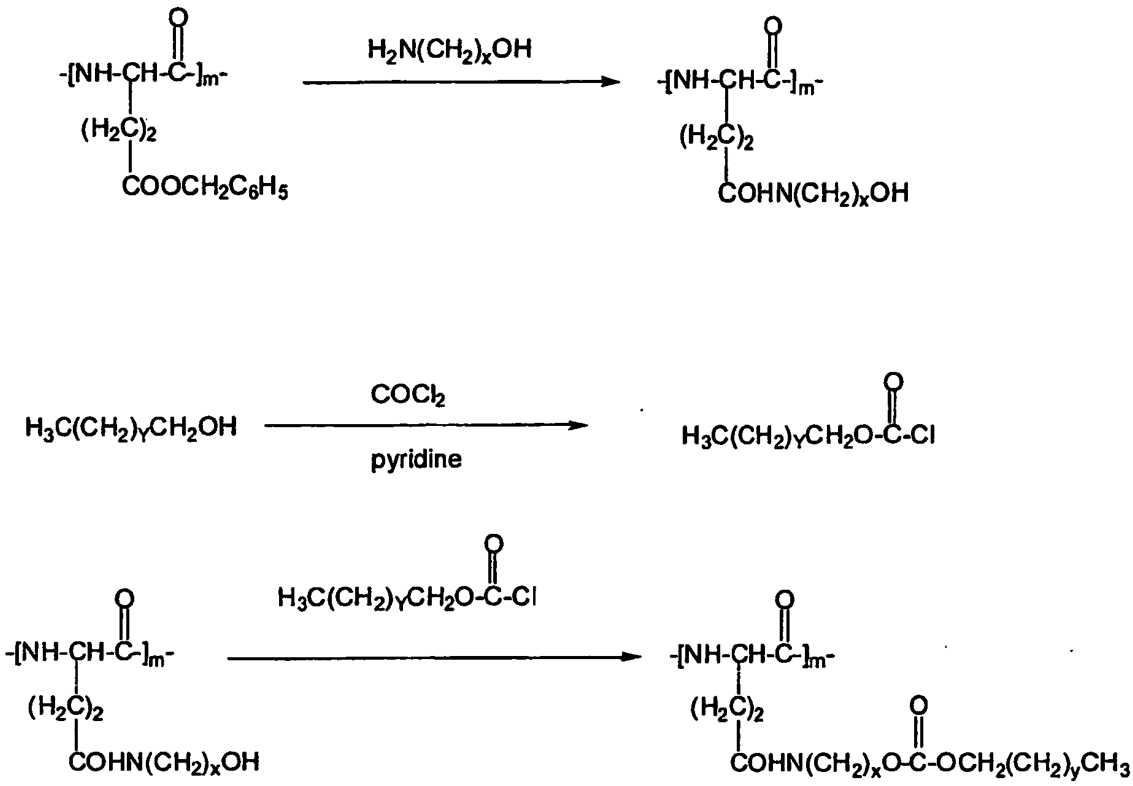


Reaction Scheme 2.

Or an oxime ester linkage may be used:

<u>Oxime Ester</u>

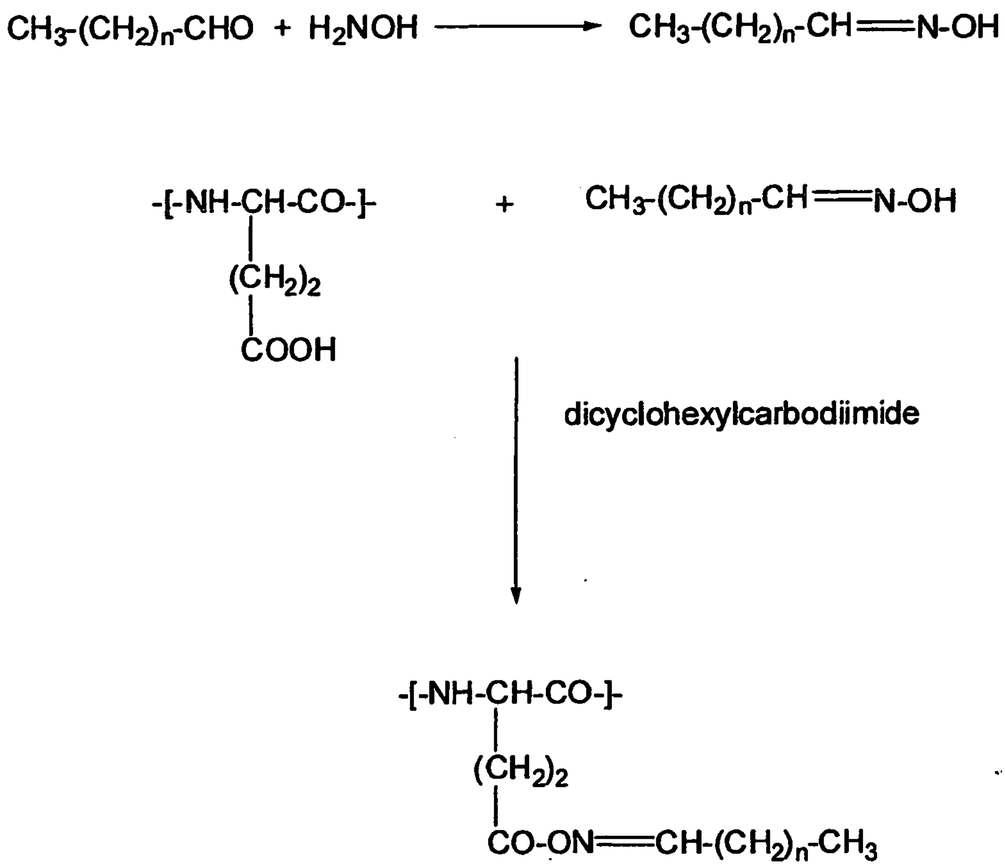


Reaction Scheme 3.

*Coatings*

Coatings are formed on an object. In contrast, other constructs, e.g., sheaths, sleeves, membranes, and molded objects, can be manufactured separately from a particular device. Consequently, coatings are distinct from other types of polymeric construct. For example, a sleeve, sheath, or membrane requires a certain minimum of mechanical robustness so as to maintain its identity before being associated with an object. Further, a process of coating creates an intimacy of contact between the coating and the device that is often desirable; for this reason, some processes involve coatings instead of other manufacturing procedures. Moreover, some processes of coating an object such as spraying or dipping create physical properties or processing opportunities that are not available in other processes. Further, other teachings that are related to treatment of devices may not be applicable for coatings because of these differences.

It is recognized, however, that a coating can have variable characteristics. Thus a coating may be discontinuous with a surface at some points and still retain its characteristic as a coating. Coatings may also be formed of a single layer, or a plurality of layers. Coatings, and layers, can have a variable thickness, variable composition, variable chemical properties. Coatings, and layers, may cover all or a portion of a surface. Layers may, e.g., be superimposed upon other layers to create a coating.

Processes for forming a layer on an object, e.g., a medical device, may include applying a composition to a device by spraying, or by dipping the device into a composition for forming a polymeric layer. These and other methods are generally known to persons of ordinary skill in these arts. Derivatized polypeptides taught herein may be formed in layers upon a medical device, including a layer that covers all of a device, a layer that covers a portion of the device, and layers upon other layers. Layers that contact each other may be crosslinked to each other, e.g., by covalent crosslinks between polymers in the layers.

Some embodiments of layers are formed by preparing a composition of derivatized polypeptides and applying them to a surface. Other embodiments are layers formed by applying a composition of reactive derivatized polypeptides bearing functional groups to a device or a layer and initiating polymerization or other reaction of the functional groups to form a layer. Similarly, derivatized polypeptide may be applied to a device or layer and reacted there to form a layer.

Layers may also be crosslinked together. One method is to apply a first layer that has a first set of reactive functional groups, and to apply a second layer that has a second set of reactive functional groups that form covalent crosslinks with the first set of functional groups. The first layer and second layers may be applied in any order, e.g., starting with the first, then the second, or vice versa. Additional layers may be similarly formed and used.

Layers may be made from a single type of derivatized polypeptides, a plurality of derivatized polypeptide types, in combination with another compound, e.g., a polymer, or a combination thereof. For example, a single type of derivatized polypeptide could be used, or a plurality of derivatized polypeptide, each prepared separately, could be used. Or a single reactive derivatized polypeptide could be mixed with reactable or unreactable polymers.

Some layers are useful for providing a base layer that contacts a device and serves to anchor subsequently applied layers. For example, a first layer with reactive functional groups may be applied to a device, and a subsequent layer may be crosslinked to the base layer.

Some layers are formed by chemically reacting other layers, e.g., using surface chemistry. For example, a layer may have reactive functional groups that are exposed to a chemical composition of polymers or non-polymers that have a functional group to react thereto. Or, for example, a layer may be exposed to reactive functional groups that are reactable thereto. For example, a layer may be exposed to a composition of light-activatable molecules that are triggered by light to react with the layer. Or a layer having nucleophilic groups may be exposed to a composition of molecules having electrophilic groups that react with the nucleophiles.

Any of these layers may be associated with a therapeutic agent, and may be formed on a medical device with or without the presence of a therapeutic agent. A therapeutic agent may be associated with the components of the layer, before, during, or after its application to a device. Thus a layer and a therapeutic agent may be essentially simultaneously applied to a device. Such an application has some advantages, e.g., for ease of manufacturing. For example, a derivatized polypeptide may be associated with a therapeutic agent and the copolymer-therapeutic agent association may be applied to a device. Or, for example, a therapeutic agent may be part of a composition that is applied to a surface that is subsequently activated to form new copolymers. As indicated above, certain copolymers may advantageously be combined with a therapeutic agent to achieve delivery of the agent.

Therapeutic agents may be associated with a derivatized polypeptide before the derivatized polypeptide is applied to a device. The derivatized polypeptide may be prepared and then exposed to a solution containing a solvent for the agent. The agent and the derivatized polypeptide are allowed to interact, and the agent becomes associated with the derivatized polypeptide.

Or the therapeutic agent may be exposed to a derivatized polypeptide at essentially the same time that the agent and the copolymer are essentially simultaneously applied to a device. The agent and the copolymer could be in the same or different solvent. Nonsolvent and solvent are terms used somewhat broadly and include their strict meanings and also as including mixtures diluted with other substances. These terms are applied in light of a particular application, and are sometimes given meanings that indicate relatively good or relatively poor solvency.

Therapeutic agents also may be associated with a layer after the layer is applied to a device. For example, one suitable method comprises exposing the layer to a mixture comprising the agent. The mixture may further comprise a relatively good solvent for both the agent and the layer so that the layer is swelled and the agent migrates therethrough. When the solvent is removed, the agent, or at least a portion thereof, remains in the layer.

Combinations of the above methodologies for associating the coating to the device can be used for the incorporation of a single therapeutic agent or a combination of therapeutic agents.

The application of a coating to a medical device may be adapted to the particular circumstances for that device. For example, with regards to thickness, the particular application may indicate what is suitable. A stent, for example, can be threaded through a tortuous system of blood vessel to reach its point of delivery in a patient. So a coating on the stent should have suitable physical properties and thickness. The thickness of the polymeric layer is of a range that a therapeutic dose is delivered without impeding the effects of the drug and the performance of the medical device, for example a stent may have a polymeric layer in the range of, e.g., about 0.01 $\mu$m to about 150 $\mu$m, or between about 1 and about 300 $\mu$m. Other ranges for other medical devices may vary widely, but some ranges are less than about 3 mm, less than about 1 mm, less than about 0.1 mm, less than about 0.01 mm, from about 1 to about 100 $\mu$m, from about 10 to about 1000 $\mu$m, from about 1 to about 10,000 $\mu$m, and from about 10 to about 500 $\mu$m; persons of ordinary skill in these arts will realize that all values and ranges within these explicit ranges are contemplated, and that other ranges may be suited as depending upon the device and/or application.

Some devices and applications require an expandable or a flexible layer. As set forth in the Examples, embodiments herein can provide for flexibility and/or for expandability. With respect to a stent, most designs of stents require a step of expansion upon deployment in a patient. A layer that is expandable to accommodate the stent deployment is advantageous. With respect to a medical balloon, its use in the patient requires a step of expansion; accordingly, a coating on such a balloon may advantageously be made so as to accommodate that expansion.

An aspect of such coatings may be the hydrophobic balance of the coating. In the case of a hydrophobic therapeutic agent mixed into a hydrophobic coating, the diffusion of the agent though the coating may control a rate of

release of the agent from the coating. The degree of hydrophobicity of the coating may be adjusted to affect the release rate. In the case of compositions as set forth herein in the above Formulas, the number of hydrophobic groups that are present correlates to the hydrophobicity of a coating made from a chemical described by one of the Formulas, with more hydrophobic groups causing an increase in hydrophobicity.

*Medical devices*

Derivatized polypeptides may be made that are able to coat a given medical device, such as a stent; are able to incorporate therapeutic agents, e.g., diagnostic agents or other materials; and/or able to biodegrade into fragments that are biocompatible and non-toxic.

Medical devices include, for example any device that is implantable, used topically or otherwise comes in contact with living tissue at least for some period of time. The devices could be made, for example, from polymers, such as catheters; from metals, such as guide-wires, stents, embolizing coils; from polymeric fabric, such as vascular grafts, stent grafts; from ceramics, such as mechanical heart valves, or a combination of these materials. Other devices include, for example, heart valves, implantable cardiovascular defibrillators, pacemakers, surgical patches, patches, wound closure, micro-spheres, biosensors, sensors (implantable, ex-vivo and analyzers) ocular implants and contact lenses; medical devices that are made from ceramic, glass; tissue engineering scaffolds. At least partially degradable medical devices are also included. Examples of at least partially degradable medical devices include stents, e.g., urethral stent, abdominal aortic aneurysm stents, vascular stents, cardiac stents, coronary stents. Other examples of at least partially biodegradable medical devices are embolizing coils, surgical patches, wound closures, ocular implants, dressings, grafts, and valves. Medical devices are also discussed in, e.g., U. S. Patent Application Nos. 5,464,650; 5,900,246; 6,214,901; 6,517,858; US 2002/0002353; and in patent applications WO 01/87342 A2; WO 03/024500.

Further embodiments include medical devices at least partially made of materials described herein, and which are at least partially degradable. For instance, a medical device may be at least partially made of a copolymer that comprises a first monomer unit and a second monomer unit, with the first monomer unit comprising an amino acid and the second monomer unit comprising an amino acid derivatized to comprise a hydrophobic hydrocarbon side chain that has a molecular weight from about 14 to about 5000. And, for instance, a medical device may be at least partially made of a polymer as set forth in Formulas 5-9. The medical device may optionally include a therapeutic agent releasable into a patient after implantation of the device into the patient. Examples of medical devices and at least partially degradable medical devices are described, above.

*Therapeutic agents*

Materials set forth herein may be associated with therapeutic agents, including, for example, drugs, imaging agents, diagnostic agents, prophylactic agents, hemostatic agents, tissue engineering agents, nitric oxide releasing agents, gene therapy agents, agents for enhancing wound healing, and bioactive agents. A therapeutic agent may be mixed with a polymer precursor that is in solution or disposed in a solvent, and the polymer may be formed. Alternatively, the therapeutic agent may be introduced after the polymer is formed or at an intermediate point in the polymer formation process. The term therapeutic agent is used to include, for example, therapeutic and/or diagnostic agents, and/or agents that are to be released from a coating.

Many examples of therapeutic agents are set forth in priority document number 60/574,250. Therapeutic agents include, for example, those as disclosed in U. S. Patent No. 6,214,901; additional embodiments of therapeutic agents, as well as polymeric coating methods, reactive monomers, solvents, and the like, are set forth in U. S. Patent Nos. 5,464,650, 5,782,908, 5,900,246, 5,980,972, 6,231,600, 6,251,136, 6,387,379, 6,503,556, and 6,517,858.

*Glass transition temperature and polymer terminology*

Certain embodiments of copolymers described herein are related to the property referred to as glass transition temperature, Tg. Tg is the temperature at which an amorphous polymer (or the amorphous regions in a partially crystalline polymer) changes from a hard and relatively brittle condition to a viscous or rubbery condition. Glass transition temperatures may be measured by methods such as differential scanning calorimetery (DSC) or differential thermal analysis. Other methodologies include volume expansion coefficient, NMR spectroscopy and refractive index. Tg is a property of a polymer.

A polymer is a molecule composed of repeated subunits. Each subunit is referred to herein as a monomeric unit. Polymers of only a few monomeric units are sometimes referred to as oligomers. The term polymer includes, for example, the meanings of homopolymer, copolymer, terpolymer, block copolymer, random copolymer, oligomer, and the like.

Some embodiments herein are directed to copolymers having certain Tg values or averages. Unless otherwise

specified, the average Tg values are to be measured directly or estimated on the basis of weight of the monomer units. An alternative method is to calculate an average by molar weight. Herein, when calculating an average Tg by weight for a composition having monomeric units, this formula is used:

$$\text{AVERAGE Tg} = \frac{(W1Tg1 + W2Tg2 + W3Tg3 + \ldots\ WnTgn)}{(W1 + W2 + W3 + \ldots\ Wn)}$$

wherein W1, W2, W3, Wn, indicate the weight (e.g., in grams) of the first, second, third, and nth monomeric unit, respectively, and Tg1, Tg2, Tg3, and Tgn are the Tgs for the homopolymers of the first, second, third, and nth monomeric unit, respectively. The Tg for a homopolymer varies with MW until about 20,000, so that a Tg for a homopolymer is customarily considered its Tg at or above about 20,000 MW. This procedure may be used to calculate the average Tg for a composition of monomeric units that are disposed in a copolymer.

It is appreciated that commonly used synthesis methods provide a distribution of polymeric weights. A set of polymers therefore has an average weight and an average distribution of those weights. Therefore some embodiments relate to a set of polymers or polypeptides having a certain average or a distribution of properties or compositions and other aspects of polymers relate to calculating the average polymer weight. One such method is the weight average molecular weight, which is calculated as follows: weigh a number of polymer molecules, add the squares of these weights, and then divide by the total weight of the molecules. The number average molecular weight is another way of determining the molecular weight of a polymer. It is determined by measuring the molecular weight of n polymer molecules, summing the weights, and dividing by n. The number average molecular weight of a polymer can be determined by, e.g., osmometry, end-group titration, and colligative properties, and various other methods exist for estimating the number average or weight average of polymers.

A polymer may include a block. A series of identical monomeric units joined together forms a block. A polymer may have no blocks, or a plurality of blocks. Blocks from a group of polymers or from one polymer may become associated with each other to form domains. Thermodynamic forces can drive the formation of the domains, with chemical attractions or repulsions between the blocks contributing to the driving force. For example, some blocks may tend to become associated with each other as a result on ion-ion interactions or hydrophobic-hydrophilic forces. Thus, in some conditions, a composition of polymers having hydrophilic blocks and hydrophobic blocks could be expected to form domains having hydrophobic blocks and domains having hydrophilic blocks. A copolymer is a polymer having at least two different monomeric units. Some copolymers have blocks, while others have random structures, and some copolymers have both blocks and regions of random copolymer bonding. Copolymers may be made from reactive monomers, oligomers, polymers, or other copolymers. Copolymer is a term that encompasses an oligomer made of at least two different monomeric units.

*Polymers, Tg, and Copolymers with monomeric units having a predetermined selected difference in Tg*

Certain embodiments herein relate to copolymers formed from monomeric units that form homopolymers that have Tgs that have a selected difference between them. Monomeric units are sometimes referred to herein as having a Tg, by which is meant the Tg of the homopolymer of about 20,000 molecular weight formed of the monomeric unit. Without being bound to a particular theory of operation, the predetermined differences set forth herein are believed to contribute to domain formation so that certain desirable polymeric properties are enhanced. One such property is enhanced association of therapeutic agents with the domains. The domain-domain interactions may create small microvoids for therapeutic agents, or may form chemical associations with the therapeutic agents, which can be bonding associations or electrostatic interactions.

Suitable predetermined Tg differences between monomeric units include at least about 30°C, at least about 50°C, and at least about 70°C. Other suitable differences in monomeric units Tgs are in the range of about 30°C to about 500°C, about 50°C to about 300°C, and about 70°C to about 200°C. Persons of ordinary skill in these arts, after reading this disclosure, will appreciate that all ranges and values within these explicitly stated ranges are contemplated.

Tg is an indirect and approximate indication of mobility of blocks or domains of a composition of copolymers. For copolymers having a non-covalent chemical or physical association with an agent, a greater mobility, or lower Tg, would be expected to result in a faster release of the agent. Other factors that affect release are the size of the agent, its chemical characteristics, and the extent of its association with the polymers around it. Some chemical characteristics are, for example, hydrophilicity, shape/size, presence of charges, and polarity. The most desirable rate of release of an agent, however, is dependent on the application. Some situations require a quick release, some require a sustained release, and some require a quick burst, followed by a sustained release. Further, a plurality of agents may be associated

with a polymer, or a layer, or a coating, so that the Tgs are adjusted to reflect the chemistries of the agents.

In addition to choosing a predetermined Tg differences for monomeric units in a copolymer, other embodiments relate to choosing sets of monomeric units with certain Tgs relate to the average Tg of the set. In some embodiments, it is advantageous to choose a particular average Tg. For instance, polymeric implants loaded with a therapeutic agent can be made with polymers or copolymers having a Tg that is close to a physiological temperature. The Tg of the monomeric units in a polymer provides an approximation of the Tg of the resultant polymer. Thus, a weighted Tg average of a composition of monomeric units may be chosen for making a copolymer having desired properties. Alternatively, other applications call for an average Tg that is suitable to achieve a change at a temperature for that application, e.g., movement form cryostorage to superheated steam, from $CO_2$ storage to oven, from freezer to boiling, from a cooler to a hot water bath, and so forth. Weighted Tg averages for copolymers and polymers as set forth herein include from about -200°C to about 500°C, from about -80°C to about 250°C, from about -20°C to about 100°C, from about 0°C to about 40°C. Persons of ordinary skill in these arts, after reading this disclosure, will appreciate that all ranges and values within these explicitly stated ranges are contemplated.

EXAMPLES

Materials: N-hydroxysuccinimide, hexanoic acid, N,N" dicyclohexyl carbodiimide, triethylamine, 2,4,6, trinitrobenzene sulphonic acid, L-glutamic acid, L-aspartic acid, poly L-lysine hydrobromide (M.W. 70,000-150,000), sodium tetraborate, polyethylene glycol monomethyl ether (F.W. 550), 1,1[1] - carbonyldiimidazole, n-butanol, n-dodecanol, n-tetradecanol, n-hexadecanol, n-octadecanol, poly n-ethyl L-glutamate (M.W. 100,000), p-toluene-sulphonic acid, poly L-glutamic acid sodium salt (M.W. 50,000-100,000), 1-bromobutane, sodium bicarbonate and all solvents used were purchased from Sigma-Aldrich Chemical Company.

Example 1

Synthesis of Poly (L-lysine) graft n-hexyl copolymers

i. N-hydroxysuccinimide ester of hexanoic acid

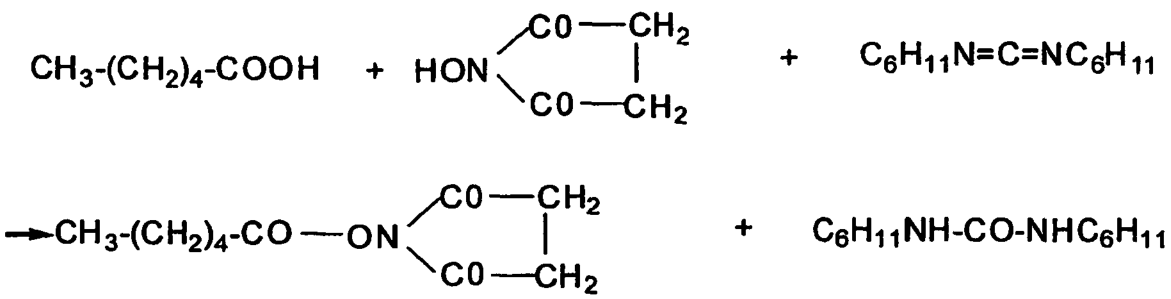

Reaction Scheme 4

N-hydroxysuccinimide ester of hexanoic acid was synthesised using the procedure described in Y. Lapidot, S. Rappoport and Y. Wolman. Journal of Lipid Research, (1967) 142-145.3.

Hexanoic acid (20g, 0.172 moles) was added to a solution of N-hydroxysuccinimide (20g, 0.172 moles) in anhydrous ethyl acetate (750ml). A solution of dicyclohexylcarbodiimide (35.49g, 0.172 moles) in anhydrous ethyl acetate (35ml) was added and the reaction mixture stirred at room temperature for 16 hours. Dicyclohexylurea was removed by filtration, and the filtrate was concentrated under reduced pressure to yield a white precipitate. The precipitate was washed with petroleum ether several times to remove dicyclohexylcarbodiimide, traces of hexanoic anhydride and hexanoic acid.

The product was dissolved in dithylether (100ml) and stored at 15°C for 16 hours. Traces of acylurea precipated and were removed byfiltration. Diethyether was removed under pressure to yield white crystals (20g, yield 50%). Thin layer chromatography (dichloromethane or petroleum ether (b.p. 40-60°C) - diethylether 8:2) gave a single spot.

ii Partial coupling of N-hydroxysuccinimide ester of hexanoic acid to amino function of poly (L-lysine)

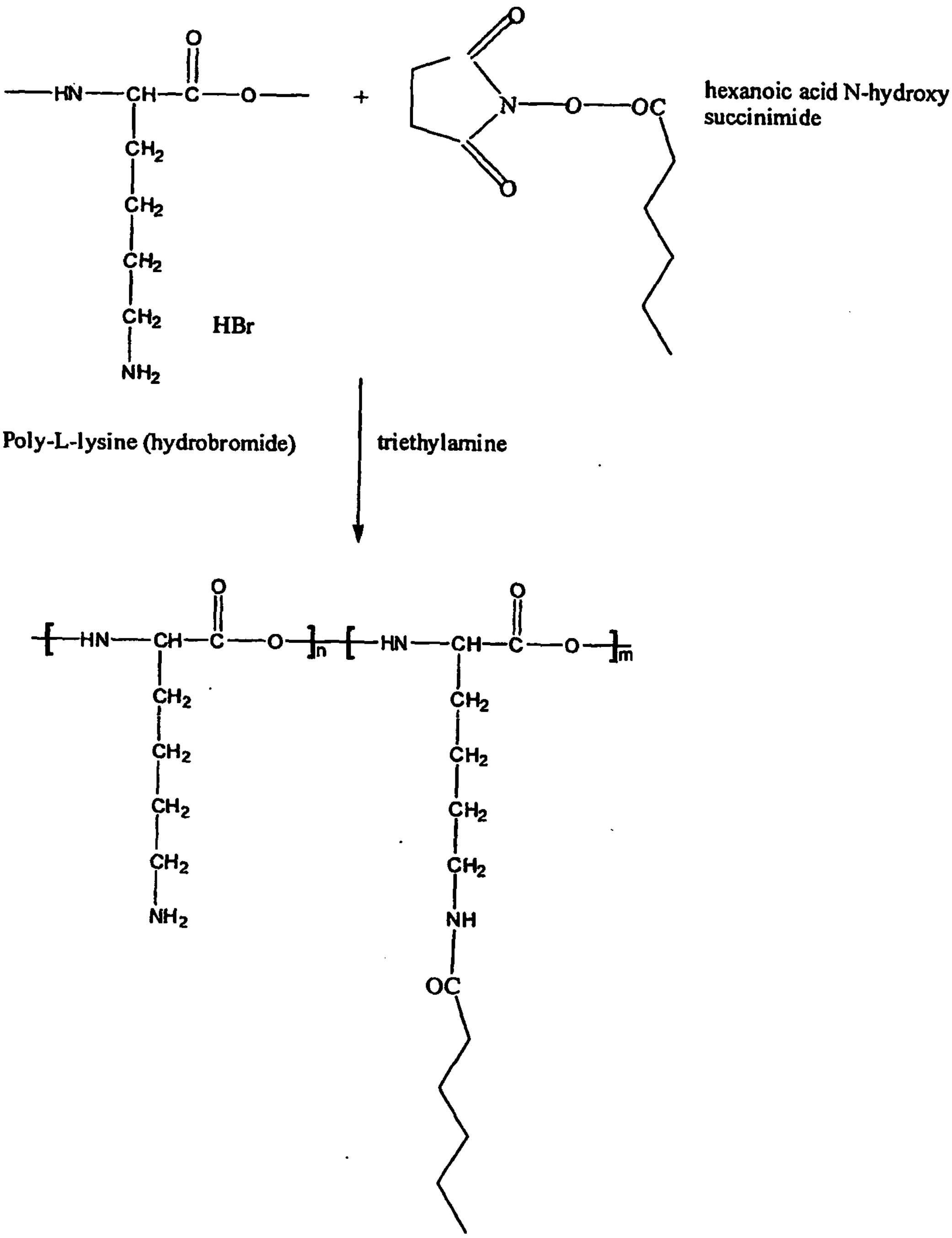


Reaction Scheme 5

In order to obtain a poly (L-lysine) graft copolymer which is soluble in organic solvents, but also has hydrophilic moieties, only some of the available amino groups were coupled to hexanoic acid.

The synthesis was performed as referenced (in D. Derrieu, P. Midoux, C. Petit, E. Negre, R. Mayer, M. Monsigny and A-C Roche. Glycoconjugate Journal 6, (1989) 241-255.) but with modifications. Poly L-lysine hydrobromide (M.W. 70,000-150,000) (2g) was dissolved in anhydrous dimethyl sulfoxide (DMSO) (50ml). To this solution N-hydroxysuccinimide ester of hexanoic acid (1.33g, 6.228 x $10^{-3}$ moles) was dissolved in anhydrous DMSO (10ml) and was added dropwise. Then triethylamine (0.628g, 6.213 x $10^{-3}$ moles) in a mixture of DMSO (8ml) and methanol (2ml) was added

dropwise to the above and allowed to stir for 1 hour. The solution was poured into acetone (600ml) and allowed to stir for 10 mins. The precipitate poly-L-lysine derivative was filtered and then re-suspended in acetone, stirred for 10 mins and filtered again and dried in a vacuum oven for 2 hours at 40°C. Yield was 2g of poly (L-lysine) graft n-hexyl copolymer.

The product was soluble in alcohols (methanol, ethanol and 2-propanol), DMSO, DMF but insoluble in water. Gel permeation chromatography (GPC) gave a M.W. range of 80,000 - 160,000.

The extent of coupling of hexanoic acid to poly L-lysine was found to be 60%. That is 60% of the available amino groups on poly L-lysine were chemically linked via an amide bond to hexanoic acid (details of the procedure are given below).

iii. Higher coupling of N-hydroxysuccinimide ester of hexanoic acid to amino functions of poly L-lysine

The product from example 1(ii) (2g) was dissolved in methanol (40ml). An excess of n-hydroxysuccinimide ester of hexanoic acid (1g, 4.69 x $10^{-3}$ moles) in methanol (20ml) was added to the methanol solution. Triethylamine (0.726g) in methanol (10ml) was added dropwise to the above over a period of 15mins. After which time, solution was stirred for 30 mins. The solution was then poured into acetone (600ml) and the product precipitated. The product was filtered and then re-suspended in acetone and filtered again.

The product was suspended in water (2L) and stirred for 16 hours and then filtered to remove any triethylamine hydrobromide. The product was dried under vacuum (40°C) for 6 hours. Yield was 2.1g. GPC gave a M.W. range of 90,000-180,000. 94% of the free amino groups in poly L-lysine were coupled to hexanoic acid via an amino bond when assayed using the TNBS assay for free amino groups (details of procedure given below).

iv. TNBS assay for free amino groups

2, 4, 6-trinitrobenzene sulphonic acid is used for the determination of free amines (S. Snyder and P. Sobocinski. Anal. Biochem, 64, (1975) 284-288). The proportion of free amino groups in the grafted poly L-lysine may be measured by a comparative titration of the free amino groups in both the unmodified and modified poly L-lysine using TNBS. The TNBS was carried out by dissolving the poly L-lysine graft n-hexyl copolymers (5mg) in methanol (10ml); 5mls of this solution was diluted to 10ml with sodium tetraborate (0.1M). TNBS solution (7.5 ul, 0.03M) was added to a 5ml aliquot of this methanolic sodium tetraborate solution. The resulting solution was mixed thoroughly and incubated at room temperature for 30min. The absorbance was read at 420nm (lambda 2, Perkin Elmer), and the weight percent of unmodified polymer was interpolated from standard curve prepared using the unmodified poly L-lysine.

Therefore, in Example 1 (ii), the percentage was 40%, hence a degree of incorporation of hexanoic acid of 60%. Similarly, for Example 1 (iii) 6% was unmodified polymer and hence 94% incorporation of hexanoic acid onto poly L-lysine.

Example 2

Synthesis of poly (L-lysine) graft n-hexyl graft polyethylene glycol copolymers

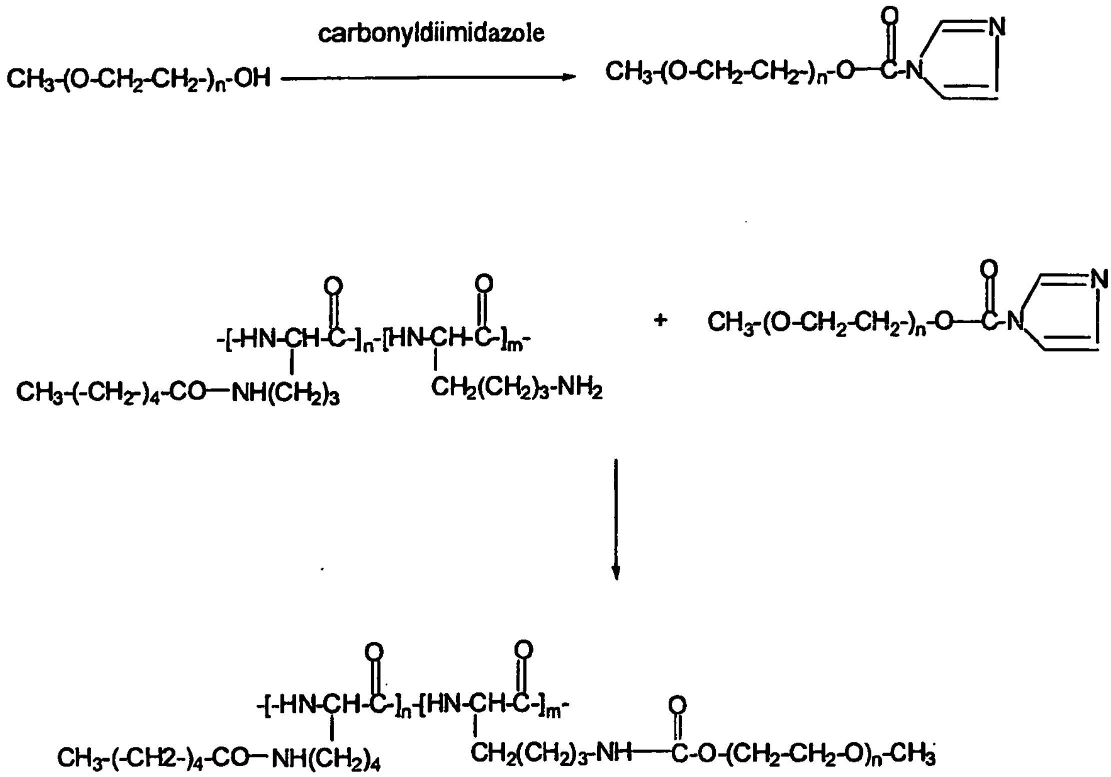


Reaction Scheme 6

The hydrophobic/hydrophilic balance can be further achieved by grafting hydrophilic polyethylene glycol onto the poly L-lysine.

From Example 1 (ii), using the partial coupling method, 85% of the free amino groups on poly L-lysine were coupled to hexanoic acid, as assayed using the TNBS procedure.

Polyethylene glycol monomethyl ether (FW 550) (10g) was dissolved in anhydrous dichloromethane (50ml) and thereto was added dropwise carbonyldiimidazole (CDI) (2.95g) dissolved in 20ml dichloromethane. The solution was allowed to stir for 3 hours and then dichloromethane evaporated under reduced pressure and the product was suspended in petroleum ether (b.p. 40-60°C) and filtered. The product was re-suspended in petroleum ether and allowed to reach boiling point and whilst still warm the product was filtered and dried in vacuum (40°C) for 2 hours. I.R. showed the disappearance of the peak at 3600 due to the hydroxyl group absorption.

Poly L-lysine graft n-hexyl copolymer (from above) (2g) was dissolved in methanol (40ml). To this was added CDI-activated polyethylene glycol monomethyl ether (1.5g) dropwise in methanol (20ml). Triethylamine (0.2ml; 1.44 x $10^{-3}$ moles) was dissolved in methanol (5ml) and added dropwise thereto above. After addition the solution was allowed to stir for 4 hours. The product was precipitated into excess diether ether, filtered and re-suspended in diethyl ether and filtered and dried in vacuum (40°C) for 2 hours.

The product was suspended in water (2L) and allowed to stir for 16 hours. The product was filtered and washed with water and dried in vacuum (40°C) for 6 hours. Yield 2.2g.

TNBS assay showed that only 5% of free amino groups present on the polymer. Therefore, 85% of the amino groups were coupled to hexanoic acid via an amide bond and 10% of the amino groups were coupled to polyethylene glycol monomethyl ether (550) via a carbamate bond.

Example 3

Graft poly (hexyl-L-lysine) with 94% incorporation of hexanoic acid (1.5g) (Example 2 (iii)) was dissolved in

propan-2-ol (50ml). To a 10ml aliquot of the polymer solution was added the active agent paclitaxel (0.06g) dissolved in tetradydrofuran (THF) (10ml). A stainless steel coronary stent (18mm) was mounted onto a rotating mandrel and air sprayed with the above solution of propan-2-ol/THF containing polymer plus paclitaxel. The coated stent was vacuum dried at 70°C for 1 hour.

Paclitaxel loading on the stent was measured by incubating a coated stent in acetonitrile (3ml), vortexing (30 seconds) and then measuring the absorbance at 227nm wavelength: drug loading was interpolated from a standard curve. Typical drug loading per stent was 100μg+/-10%.

Paclitaxel release profiles were performed in phosphate buffered saline (PBS) (0.5ml, pH 7.4) at 37°C. Readings were taken at intervals of 1 hour, 24 hours or 48 hours. Quantification of paclitaxel was performed on HPLC, using a Nucleosil TM 100-5CIS column (I.D. 150mm x 4.6mm) (Hichrom UK Ltd); mobile phase 50% water:50% acetonitrile; flow rate of 2.0ml/min; column temperature 55°C; detecting absorbance of 227nm. Figs 1 & 2 show paclitaxel release profiles at 37°C from graft poly (hexyl-L-lysine) with 94% incorporation of hexanoic acid.

Example 4

Synthesis of Poly (gamma-n-tetradecyl-L-glutamate) - (PTLG)

Synthesis of gamma-n-tetradecyl-L-glutamate - (TLG)

TLG was synthesized using the procedure detailed in D. Wasserman, Springfield, J.D. Garber, F.M. Meigs, US Patent No 3,285,953.

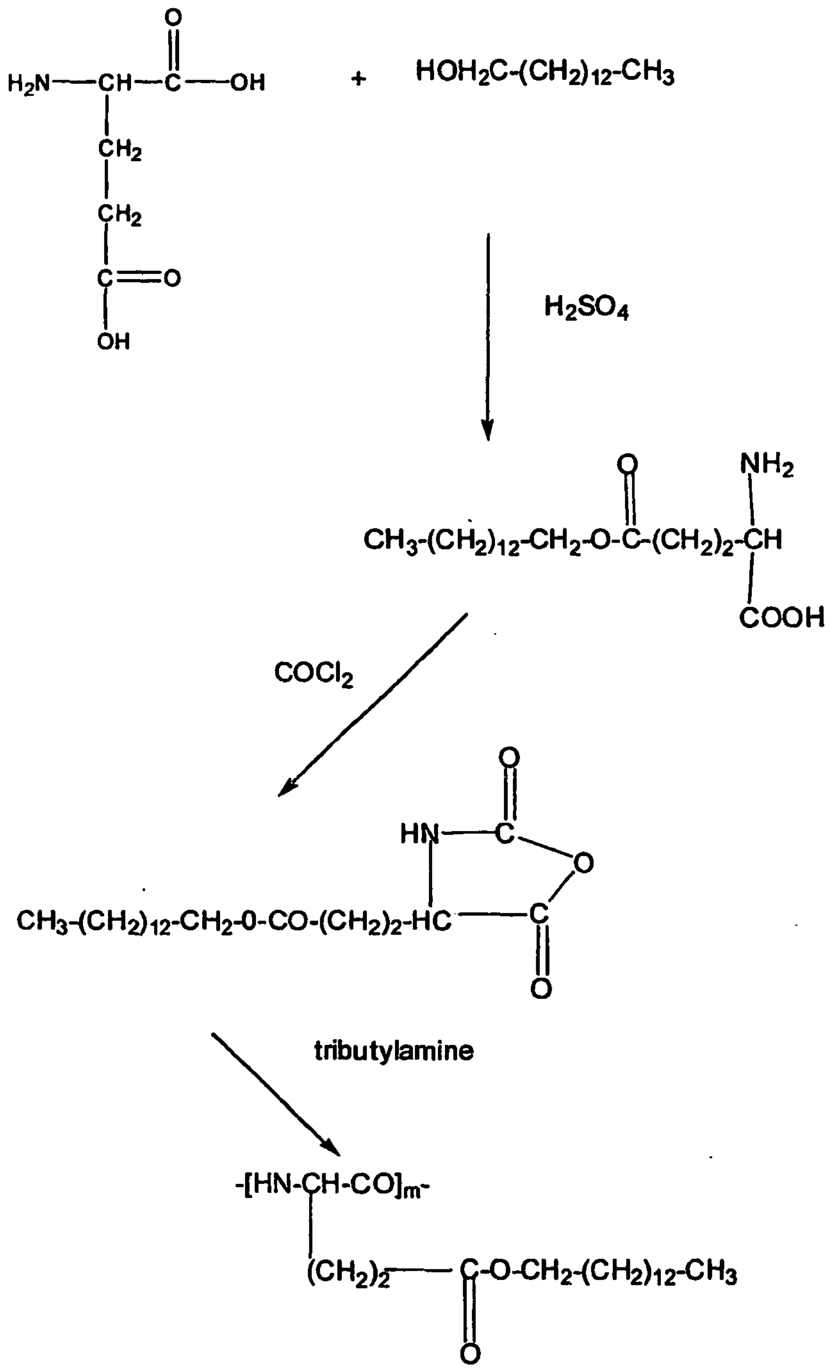


Reaction Scheme 7

A 2 liter 3-neck flask was equipped with stirrer, thermometer and additional funnel was charged with L-glutamic acid (22g, 0.15 moles), t-butyl alcohol (150ml) and n-tetradecanol (128.4g, 0.599 moles). This mixture was stirred and heated to 55°C and then sulphuric acid (97%) (12ml; 0.225 moles) was added dropwise thereto through the funnel. The temperature of the mass was then raised to and maintained at 65°C until the mass became a clear solution. The solution was maintained at 65°C for 1 hour. The heat was turned off and triethylamine (10.4ml; 0.075 moles) was added dropwise thereto to neutralise free sulphuric acid. This was followed by the addition thereto of 40ml of water and then 550ml of 95% ethanol. Whilst stirring, triethylamine (20.9ml: 0.15 moles) was added thereto.

Upon cooling to room temperature, crude free glutamate precipitated and was filtered. The recovered precipitate was slurried for 30mins at 65°C with water (500ml) and a solid cake was recovered. The cake was washed with methanol

(200ml) and then diethyl ether (200ml) and then dried at 50°C in a vacuum oven for 2 hours.

The crude precipitate was suspended in a mixture of water/2-propanol (2.5:1) and heated to 80°C and then cooled and filtered at 40°C. The recovered precipitate was washed with cold water/2-propanol mixture, then with methanol (100ml) and finally diethyl ether (100ml) and dried in a vacuum oven at 30°C to constant weight. The dry pure w-n-tetradecyl-L-glutamate weighed 15g (27% yield).

Synthesis of Poly (gamma-n-tetradecyl-L-glutamate) - (PTLG)

PTLG was synthesised from TLG via the carboxy-anhydride of TLG (W.D. Fuller, M.S. Verlander, M. Goodman. Biopolymers 15 (1976) 1869-1871). TLG (15g, 0.043 moles) was suspended in anhydrous THF (100ml) and reacted with phosgene in toluene (2M, 65ml) at 65°C for 1 hour. The solution was poured into excess petroleum ether (bp 40-60°C) and stored at -15°C for 24 hours. The precipitate was filtered and washed with petroleum ether several times to give the product gamma-n-tetradecyl-L-glutamate carboxy-anhydride, (15g, 90% yield). I.R., NCA bonds at 1762 and 1855.

Gamma-n-tetradecyl-L-glutamate carboxy-anhydride (15g) was dissolved in anhydrous dichloromethane (50ml) in a flask fitted with a condenser and drying tube. To this solution tributylamine (0.3g) was added and the mixture heated in a water bath and kept under reflux for 1 hour, following which the reaction mixture was kept at room temperature with stirring for 48 hours. Dichloromethane was evaporated under reduced pressure and then methanol (100ml) was added. The product precipitated and was filtered. The product was re-suspended in methanol (100ml) and heated to boiling and then cooled and product was then filtered and washed with methanol (100ml) and then dried in a vacuum oven at 30°C to constant weight. Yield was 10g. GPC gave a M.W. of 60,000-90,000.

Example 5

Synthesis of Poly (gamma-n-tetradecyl-L-aspartate) - (PTLA)

PTLA was synthesised exactly as described in Example 4 for the synthesis of PTLG. Yield of intermediates and product were similar but the M.W. by GPC was 90,000-110,000.

Example 6

Synthesis of Poly (gamma-n-dodecyl (PDLG), hexadecyl (PHLG) and octadecyl-L-glutamate (POLG))

Synthesis of the above polymers with chain lengths (dodecyl C12; hexadecyl C14; Octadecyl C18) were synthesised according to the procedure outlined in Example 4. These polymers had similar yields and M.W. were between 75,000-115,00. Someone trained in the art may also produce a polymer with all three chain lengths and more on the same polymer backbone.

Example 7

Synthesis of random copolymers (gamma-n-tetradecyl-L-aspartate and gamma-n-teratdecyl-L-glutamate)

The synthesis of a random copolymer of L-aspartate and L-glutamate with both amino acids having hydrophobic chains (n-tetradecyl) was accomplished using the procedure set out in Example 4. GPC revealed a M.W. distribution of 30,000-70,000.

Example 8

Synthesis of Poly (gamma-n-butyl-L-glutamate) - (PBLG) from Poly (gamma-n-ethyl-L-glutamate) - (PELG) via transesterification.

PELG (1g) was dissolved in anhydrous 1,2,dichloroethane with heating. The dissolved PELG gave a cloudy solution. 1-Butanol (4g) was added thereto together with p-toluene sulphonic acid (0.3g). The mixture was refluxed at 80°C for 24 hours. At which point a clear solution resulted. 1,2,dichloroethane was evaporated under reduced pressure and then the product was suspended in methanol (50ml). A white precipitate resulted which was filtered and re-suspended in methanol (50ml) and re-filtered and washed with methanol and dried in a vacuum oven at 30°C to constant weight. Yield (1g). The product PBLG was solvent in chlorinated solvents and THF whereas PELG only gave cloudy solutions in chlorinated solvents. $^{1}$HNMR showed that the degree of substitution of butyl for ethyl was 96%. GPC gave a M.W.

distribution of 115,000-130,000.

Example 9

An example of increasing the hydrophilic nature of poly-n-alkyl glutamates is to partially esterify n-alkyl groups onto polyglutamic acid. Polyglutamic acid with sodium salt (M.W. 50,000-100,000) (1g) was dissolved in water (50ml) and thereto was added hydrochloric acid (0.1M) dropwise until the pH of the solution was 1.5. This was dialysed against water (10L) using Cellu Sep membrane M.W.C.O. 12,000-14,000 for 24 hours and freeze dried. Yield 0.7g. The partial synthesis of poly-n-alkyl glutamate was performed as described in T. Shimokuri, T. Kaneko, T. Serizawa and M. Akashi, Macromolecular Bioscience 4 (2004) 407-4116.

Polyglutamic acid (0.7g, 5mmol) was suspended in N-methyl pyrolidone (50ml) and then sodium bicarbonate (0.84g, 10mmol) was added thereto and stirrerd for 2 hours at 60°C. 1-Bromobutane (1.03g, 7.5mmol) was then added. The solution was stirred for 24 hours at 60°C. Precipitated sodium bromide was filtered off and the reaction mixture was poured into methanol (400ml). The filtered product was again suspended in methanol (200ml) and filtered and vacuum dried at 40°C for 2 hours. The dried product was suspended in water (2L) and stirred 16 hours. The product was filtered and dried in a vacuum oven (40°C) for 6 hours.

The esterification was confirmed by $^1$HNMR. The degree of esterification as measured by $^1$HNMR spectroscopy was 75%. The polymer was insoluble in water but soluble in chloroform, benzene, DMF and DMSO. Someone skilled in the art may also produce partial esters of poly alkyl - L aspartate or a mixture of partial esters of poly alkyl-L-glutamate and poly alkyl-L-aspartate. This known procedure serves equally well for poly-n-alkyl-gamma-glutamate where one can have partial or total esterification depending on the molar ratio of the bromo alkyl used (T. Shimokuri, T. Kaneko, T. Serizawa and M. Akashi, Macromolecular Bioscience 4 (2004) 407-411).

Example 10

Poly (gamma-n-butyl L-glutamate) (0.15g) was dissolved in anhydrous THF (20ml). Thereto was added paclitaxel (0.04g) and stirred until dissolved. A stainless steel coronary stent (12mm) was mounted onto a rotating mandrel and air sprayed with the above solution. The coated stent was then vacuum dried at 70°C for 1 hour. This was similarly carried out for Poly (gamma-n-tetradecyl-L-glutamate). But for Poly (gamma n-ethyl L-glutamate), the polymer was dissolved in 1,2 dichloroethane to give a cloudy solution.

Paclitaxel loading on the stents was measured by incubating a coated stent in acetonitrile (3ml), vortexing (30 seconds) and then measuring the absorbsance at 227nm wavelength. Drug loading was interpolated from a standard curve. Typical drug loading per stent was 120μg +/-10%.

Paclitaxel release profiles were performed in PBS (0.5ml, pH 7.4) at 37°C. Readings were taken at intervals of 1 hour, 24 hours or 48 hours. Quantification of paclitaxel was performed on HPLC, using a nucleosil TM100-5CIS column (I.D. 150mm x 4.6mm) (Hichrom UK Ltd); mobile phase 50% water:50% acetonitrile; flow rate of 2.0ml/min; column temperature 55°C; detecting absorbance of 227nm.

Drug release (paclitaxel) and control of drug release is exemplified in Figs 3 & 4. Paclitaxel release from three poly gamma-n alkyl L-glutamates with different chain lengths is shown. Modulation of paclitaxel release is achieved by varying the chain length. In this instance the longer the chain length, the greater the release of paclitaxel.

Example 11

*in vitro* degradation of poly-L-lysine derivatives was performed on thin films of polymer cast onto PTFE sheets. The films were dried and then circular films were cut to a defined diameter (50mm) using a cork -borer. The films were further dried to a constant weight (90-100mg).

The circular films were incubated with 10ml of solution in glass test tubes incubated at 37 °C. The incubation solutions were: .

Phosphate buffer (200mM) pH 7.4
Phospate buffer plus 5mg of α-chymotrypsin.

The above solutions were changed every 24 hours. The films were removed from the incubating solution and washed with deionised water and dried to a constant weight.

Table 1: Summary of the results obtained.

| Polymer | *in vitro* biodegradation in Phosphate Buffer (% weight loss in 14 days) | *in vitro* biodegradation in Phosphate Buffer + α-chymotrypsin (% weight loss in 14 days) |
|---|---|---|
| Poly(n-hexyl-L-lysine) 65% coupling (from Example 1 ii) | 2.1% | 7.6% |
| Poly(n-hexyl-L-lysine) 94% coupling (from Example 1 iii) | 0% | 4.3% |
| Poly(n-hexyl-L-lysine)co polyethylene glycol monomethylether-L-lysine (from Example 2) | 2.2% | 6.4% |

Example 12

In-vitro degradation of Poly-L-glutamate or aspartate derivatives was performed exactly as described in Example 11. The table below (Table 2) summarizes the results obtained.

Table 2

| Polymer | *in vitro* biodegradation in Phosphate Buffer (% weight loss in 14 days) | *in vitro* biodegradation in Phosphate Buffer + α-chymotrypsin (% weight loss in 14 days) |
|---|---|---|
| PELG | 3.1% | 8.2% |
| PBLG | 0% | 6.1% |
| PDLG | 0% | 3.9% |
| PTLG | 0% | 4.0% |
| PHLG | 0% | 4.2% |
| POLG | 0% | 2.1% |
| Poly(gamma-n-tetradecyl-L-aspartate) co(gamma-n-tetradecyl-L-glutamate) | 0% | 4.1 % |
| Poly(gamma-n-butyl-L-glutamate) (Partial esterification from Example 9) | 3% | 8.1% |

## Claims

1. A method of making biodegradable coating on a surface of a medical device comprising:

    forming a biodegradable layer on at least a portion of the surface of the medical device, the layer comprising a copolymer and a therapeutic agent releasable into a patient after implantation of the device into the patient,

    wherein the copolymer comprises a first monomer unit and a second monomer unit, with the first monomer unit comprising an amino acid and the second monomer unit comprising an amino acid derivatized to have a hydrophobic hydrocarbon side chain that has a molecular weight from 14 to 5000.

2. The method of claim 1 wherein the medical device is degradable after implantation into a patient.

3. The method of claim 1 wherein the hydrophobic hydrocarbon side chain comprises an alkyl chain.

4. The method of claim 1 wherein the copolymer is made by reacting side chains of a polymer with hydrophobic hydrocarbons moietics to form the hydrophobic hydrocarbon side chains of the copolymer.

5. The method of claim 4 wherein 3% to 100% of the side chains of the polymer are derivatized with the hydrophobic hydrocarbons.

6. The method of claim 4 wherein the polymer is a homopolymer.

7. The method of claim 1 wherein the copolymer further comprises hydrophilic side chains.

8. The method of claim 7 wherein the hydrophilic side chains comprise polyethylene oxides, sugar residues, or polysaccharides.

9. The method of claim 7 wherein the hydrophilic side chains comprise functional groups.

10. The method of claim 7 wherein the functional groups are chosen from the group consisting of hydroxyl, carboxyl, zwitterionic, suphonate, phosphate, and amino.

11. The method of claim 1 further comprising selecting the glass transition temperature of the copolymer by adjusting a number or length of the hydrophobic hydrocarbon side chains in the copolymer.

12. The method of claim 1 further comprising selecting the rate of release of the therapeutic agent from the layer by adjusting a number or length of the hydrophobic hydrocarbon side chains in the copolymer.

13. The method of claim 1 further comprising selecting the solubility of the copolymer in an organic solvent by adjusting a number or length of the hydrophobic hydrocarbon side chains in the copolymer.

14. The method of claim 1 wherein a chemical group connecting the amino acid side chain and the hydrophobic hydrocarbon comprises an amide, ester, carbonate, carbamate, oxime ester, acetal, ketal, urethane, urea, enol ester, oxazolindies, or anhydride.

15. The method of claim 1 wherein the copolymer comprises a derivatized gamma-polyamino acid.

16. The method of claim 15 wherein the gamma-polyamino acid comprises polyglutamic acid, polyaspartic acid, or polylysine.

17. The method of claim 1 wherein the therapeutic agent is entrapped in the layer or covalently linked to the copolymer.

18. The method of claim 1 wherein the copolymer is soluble in an organic solvent and is insoluble in aqueous solution.

19. The method of claim 1 wherein the first amino acid comprises a derivatized side chain.

20. The method of claim 1 wherein 5% to 95% of all of the amino acids in the copolymer comprise a derivatized side chain.

21. The method claim 1 wherein at least a portion of the copolymer comprises a formula of:

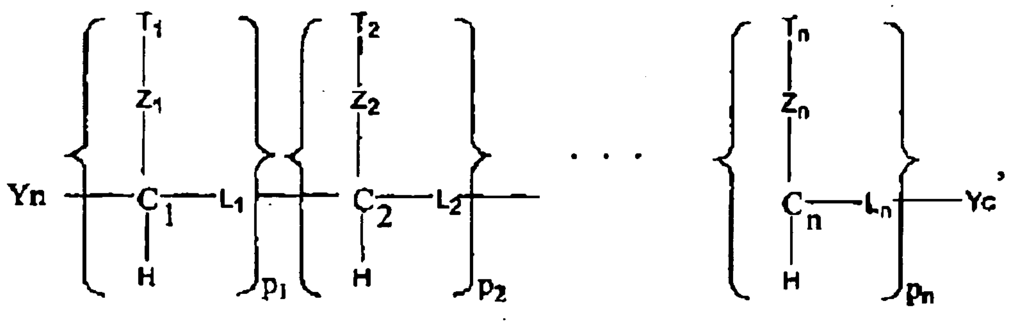

Wherein:

p is an integer that indicates a monomer unit of the polymer, and $p_1$ indicates the first monomer unit and $p_2$ indicates the second monomer unit;
n is an integer between 1 and 500,000;
$T_1$... Tn are independently chosen to have a formula of H or $-(CH2)_m-X^*$ group,
wherein X* is a H, a halogen, a hydroxyl group, a thiol group, a carboxyl group, an amino group, an alkyl group, an alkoxy group, an alkenyl group, an alkynyl group, and $-(CH2)_m$ is a group where m is an integer between, 1 and 100, inclusive, and one or more of the methylene groups is optionally replaced by O, S, N, C, C=O, an $NR_a$ group, a $CR_b$ group, a $CR_cR_d$ group, or a $SiR_eR_f$ where $R_a$, $R_b$, $R_c$, $R_d$, $R_e$, and $R_f$ are, each independently, a bond, a pi bond, H, a hydroxyl group, a thiol group, a carboxyl group, a carbamate, an oxocarbon group, an amino group, an amido group, an amide group, a phosphate group, a sulfonate group, an alkyl group, an alkoxy group, an alkenyl group, an alkynyl group, a siloxane or a functional group;

wherein the hydrophobic hydrocarbon side chain has a molecular weight from 14 to 5000;
$L_1$ ... $L_n$ are independently chosen to be a bond, or a linking group that links the indicated carbons by covalent bond(s);
$Z_1$... $Z_n$ are independently chosen to be a bond or a linking group of less than 100 atoms;
and Yc and Yn are each independently chosen to be H, a halogen, a hydroxyl group, a thiol group, a carboxyl group, an amino group, an alkyl group, an alkoxy group, an alkenyl group, an alkynyl group, a siloxane or a functional group.

**22.** A biodegradable coating on a surface of a medical device comprising:

a biodegradable layer on at least a portion of the surface of the medical device, the layer comprising a copolymer and a therapeutic agent releasable into a patient after implantation of the device into the patient, wherein the copolymer comprises a first monomer unit and a second monomer unit, with the first monomer unit comprising an amino acid and the second monomer unit comprising an amino acid derivatized to comprise a hydrophobic hydrocarbon side chain that has a molecular weight from, 14 to 5000.

**23.** The coating of claim 22 wherein the medical device is degradable after implantation into a patient.

**24.** The coating of claim 22 wherein the hydrophobic hydrocarbon side chain comprises an alkyl chain.

**25.** The coating of claim 22 wherein the first monomer unit and the second monomer unit are part of a polyamino acid derivatized to form at least a portion of the copolymer.

**26.** The coating of claim 25 wherein the polyamino acid comprises a homopolymer.

**27.** The coating of claim 25 wherein the glass transition temperature of the polyamino acid is changed by at least about 10 degrees Centigrade by the addition of the hydrophobic hydrocarbons to the polyaminoacid.

**28.** The coating of claim 25 wherein the rate of release of the therapeutic agent of the polyamino acid is changed by the addition of the hydrophobic hydrocarbons to the polyaminoacid.

**29.** The coating of claim 25 wherein the hydrophobic hydrocarbons on the polyamino acid impart solubility in organic solvents to the polyamino acid, wherein the polyamino acid is effectively insoluble in the organic solvent without the

hydrophobic hydrocarbons.

**30.** The coating of claim 22 wherein 3% to 100% of the side chains of the copolymer are derivatized with the hydrophobic hydrocarbons.

**31.** The coating of claim 22 wherein the copolymer further comprises hydrophilic side chains.

**32.** The coating of claim 31 wherein the hydrophilic side chains comprise polyethylene oxides, sugar residues, or polysaccharides.

**33.** The coating of claim 31 wherein the hydrophilic side chains comprise functional groups.

**34.** The coating of claim 33 wherein the functional groups are chosen from the group consisting of hydroxyl, carboxyl, zwitterionic, suphonate, phosphate, and amino.

**35.** The coating of claim 22 wherein a chemical group connecting the amino acid side chain and the hydrophobic hydrocarbon comprises an amide, ester, carbonate, carbamate, oxime ester, acetal, ketal, urethane, urea, enol ester, oxazolindies, or anhydride

**36.** The coating of claim 22 wherein the copolymer comprises a derivatized gamma-polyamino acid.

**37.** The coating of claim 36 wherein the gamma-polyamino acid comprises polyglutamic acid, polyaspartic acid, or polylysine.

**38.** The coating of claim 22 wherein the therapeutic agent is entrapped in the layer or covalently linked to the copolymer.

**39.** The coating of claim 22 wherein the copolymer is soluble in an organic solvent and is insoluble in aqueous solution.

**40.** The coating of claim 22 wherein the first amino acid comprises a derivatized side chain.

**41.** The coating of claim 22 wherein at least a portion of the copolymer comprises a formula of:

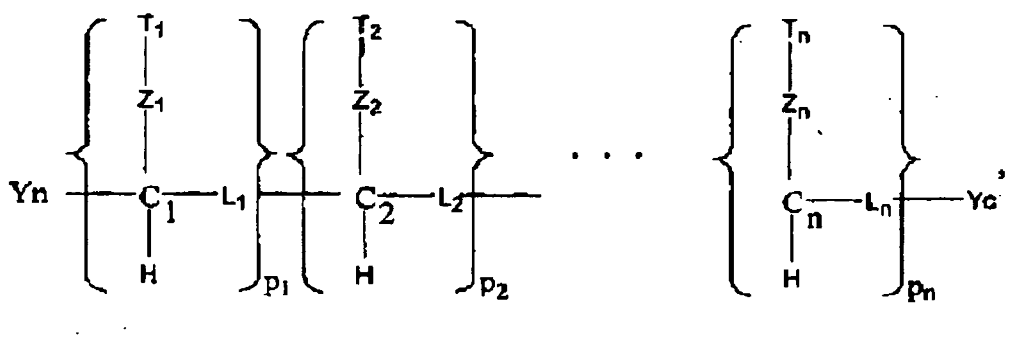

Wherein:

p is an integer that indicates a monomer unit of the polymer, and $p_1$ indicates the first monomer unit and $p_2$ indicates the second monomer unit;
n is an integer between 1 and 500,000;
$T_1$... Tn are independently chosen to have a formula of H or $-(CH2)_m-X^*$ group,
wherein X* is a H, a halogen, a hydroxyl group, a thiol group, a carboxyl group, an amino group, an alkyl group, an alkoxy group, an alkenyl group, an alkynyl

group, and $-(CH2)_m$ is a group where m is an integer between, 1 and 100, inclusive, and one or more of the methylene groups is optionally replaced by O, S, N, C, C=O, an $NK_a$ group, a $CR_b$ group, a $CR_cR_d$ group, or a $SiR_eR_f$ where $R_a$, $R_b$, Rc, $R_d$, $R_e$, and $R_f$ are, each independently, a bond, a pi bond, H, a hydroxyl group, a thiol group, a carboxyl

group, a carbamate, an oxocarbon group, an amino group, an amido group, an amide group, a phosphate group, a sulfonate group, an alkyl group, an alkoxy group, an alkenyl group, an alkynyl group, a siloxane or a functional group; wherein the hydrophobic hydrocarbon side chain has a molecular weight from 14 to 5000;
$L_1$ ... $L_n$ are independently chosen to be a bond, or a linking group that links the indicated carbons by covalent bond(s);
$Z_1$...$Z_n$ are independently chosen to be a bond or a linking group of less than 100 atoms;
and Yc and Yn are each independently chosen to be H, a halogen, a hydroxyl group, a thiol group, a carboxyl group, an amino group, an alkyl group, an alkoxy group, an alkenyl group, an alkynyl group, a siloxane or a functional group.

**42.** A medical device operable to deliver a therapeutic agent, the medical device comprising a coating according to any one of claims 22, 24 - 41.

**43.** The medical device of claim 42 wherein the coating comprises a layer of the therapeutic agent and water soluble polyamino acids on at least a portion of a surface of the medical device; the polyamino acids having been crosslinked to each other whereby to stabilize the layer, wherein between 1% and 100% of the side chains of the polyamino acids are derivatized to have a hydrophobic hydrocarbon side chain that has a molecular weight from 14 to 5000.

**44.** The medical device according to claim 43 wherein the polyamino acids have been crosslinked to each other upon curing the layer with an application of heat or ultraviolet energy.

**45.** The medical device according to claim 42 wherein the medical device is degradable after implantation into a patient.

**Patentansprüche**

**1.** Eine Methode zur Erstellung einer biologisch abbaubaren Beschichtung auf der Oberfläche eines Medizinproduktes:

bilden einer biologisch abbaubaren Schicht auf mindestens einem Teilbereich der Oberfläche des Medizinproduktes,

wobei die Schicht aus einem Copolymer und einem Therapeutikum, welches nach Implantation des Medizinproduktes in einen Patienten in den Patienten freigegeben wird, besteht,
wobei das Copolymer aus einem ersten Monomerbaustein und einem zweiten Monomerbaustein besteht, wobei der erste Monomerbaustein eine Aminosäure und der zweite Monomerbaustein eine derivatisierte Aminosäure aufweist, um eine hydrophobe Kohlenwasserstoffseitenkette mit einem Molekulargewicht von 14 bis 5.000 zu haben.

**2.** Die Methode nach Anspruch 1, wobei das Medizinprodukt nach der Implantation in einen Patienten biologisch abbaubar ist.

**3.** Die Methode nach Anspruch 1, wobei die hydrophobe Kohlenwasserstoffseitenkette eine Alkylkette enthält.

**4.** Die Methode nach Anspruch 1, wobei das Copolymer durch Reaktion der Seitenketten eines Polymers mit hydrophoben Kohlenwasserstoffresten zur Bildung der hydrophoben Kohlenwasserstoffseitenkette des Copolymers gebildet wird.

**5.** Die Methode nach Anspruch 4, wobei 3% bis 100% der Seitenketten des Polymers mit den hydrophoben Kohlenwasserstoffen derivatisiert sind.

**6.** Die Methode nach Anspruch 4, wobei das Polymer ein Homopolymer ist.

**7.** Die Methode nach Anspruch 1, wobei das Copolymer hydrophobe Seitenketten aufweist.

**8.** Die Methode nach Anspruch 7, wobei die hydrophoben Seitenketten Polyethylenoxide, Zuckerreste oder Polysaccharide enthalten.

**9.** Die Methode nach Anspruch 7, wobei die hydrophoben Seitenketten Funktionsgruppen enthalten.

**10.** Die Methode nach Anspruch 7 wobei, die Funktionsgruppen aus den Gruppen Hydroxyl, Carboxyl, zwitterionisch, Suphonat, Phosphat und Amino gewählt werden.

**11.** Die Methode nach Anspruch 1, wobei die Glasübergangstemperatur des Copolymer durch Anpassung der Anzahl oder Länge der hydrophoben Kohlenwasserstoffseitenketten im Copolymer eingestellt wird.

**12.** Die Methode nach Anspruch 1, wobei die Freigabegeschwindigkeit des Therapeutikums aus der Schicht durch Anpassung der Anzahl oder Länge der hydrophoben Kohlenwasserstoffseitenketten im Copolymer eingestellt wird.

**13.** Die Methode nach Anspruch 1, wobei die Löslichkeit des Copolymer in einem organischen Lösungsmittel durch Anpassung der Anzahl oder Länge der hydrophoben Kohlenwasserstoffseitenketten im Copolymer eingestellt wird.

**14.** Die Methode nach Anspruch 1, wobei eine chemische Gruppe, die die Aminosäurenseitenkette und den hydrophoben Kohlenwasserstoff verbindet ein Amid, Ester, Carbonat, Carbamat, Oximester, Acetal, Tetal, Urethan, Urea, Enolester, Oxazolinde oder Anhydrid enthält.

**15.** Die Methode nach Anspruch 1, wobei das Copolymer eine derivatisierte Gamma-Polyaminosäure enthält.

**16.** Die Methode nach Anspruch 15, wobei die Gamma-Polyaminosäure Polyglutaminsäure, Polyasparaginsäure oder Polylysine enthält.

**17.** Die Methode nach Anspruch 1, wobei das Therapeutikum in der Schicht eingeschlossen oder kovalent an das Polymer gebunden ist.

**18.** Die Methode nach Anspruch 1, wobei das Copolymer in einem organischen Lösungsmittel löslich und in wässriger Lösung unlöslich ist.

**19.** Die Methode nach Anspruch 1, wobei die erste Monomerbaugruppe eine derivatisierte Seitenkette enthält.

**20.** Die Methode nach Anspruch 1, wobei 5% bis 95% aller Aminosäuren im Copolymer eine derivatisierte Seitenkette enthalten.

**21.** Die Methode nach Anspruch 1, wobei mindestens ein Anteil des Copolymers folgende Formel aufweist:

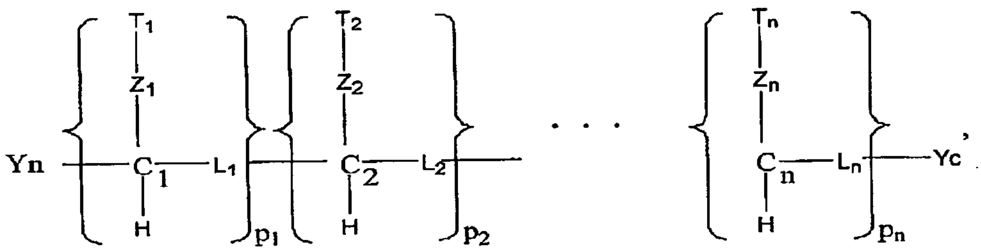

wobei:

p eine Ganzzahl ist, die einen Monomerbaustein des Polymers anzeigt, und $p_1$ ist der erste Monomerbaustein und $p_2$ ist der zweite Monomerbaustein;
n eine Ganzzahl zwischen 1 und 500.000 ist;
$T_1 ... T_n$ unabhängig ausgewählt sind und eine Formel H oder $-(CH2)_m-X^*$ Gruppe haben,
wobei X* ein H, ein Halogen, eine Hydroxylgruppe, eine Thiolgruppe, eine Carboxylgruppe, eine Amonigruppe, eine Alkylgruppe, eine Alkoxygruppe, eine Alkenylgruppe ist, und $-(CH2)_m-$ ist eine Gruppe, wo m eine Ganzzahl zwischen 1 und 100 einschließlich ist, und eine oder mehrere Methylengruppen wahlweise durch O, S, N, C, C=O, eine $NR_a$ Gruppe, eine $CR_b$ Gruppe, eine $CR_cR_d$ Gruppe oder ein $SiR_eR_f$ ersetzt werden, wobei $R_a$, $R_b$, $R_c$, $R_d$, $R_e$ und $R_f$, jeweils für sich, eine Bindung, eine pu Bindung H, eine Hydroxylgruppe, eine Thiolgruppe, eine Carboxylgruppe, ein Carbamat, eine Oxocarbon-Gruppe, eine Aminogruppe, eine Amidogruppe, eine Amidgruppe, eine Phosphat-gruppe, eine Sulfonatgruppe, eine Alkylgruppe, eine Alkoxygruppe, eine Alkenylgruppe, eine Alkynylgruppe, ein Siloxane oder andere Funktionsgruppe sind;
wobei die hydrophobe Kohlenwasserstoffseitenkette ein Molekulargewicht von 14 bis 5000 hat;
$L_1 ... L_n$ sind wahlweise ausgewählt eine Verbindung, wie eine Verbindungsgruppe, die die angezeigten Kohlenstoffe mit kovalenter(n) Bindung(en) verbindet; $Z_1$... $Z_n$ sind wahlweise ausgewählte Verbindungen oder Verbindungsgruppen mit weniger als 100 Atomen; und $Y_c$ und $Y_n$ sind jeweils unabhängig gewählt H, ein Halogen, eine Hydroxylgruppe, eine Thiolgruppe, eine Carboxylgruppe, eine Aminogruppe, eine Alkylgruppe, eine Alkoxygruppe, eine Alkenylgruppe, eine Alkynylgruppe, ein Siloxane oder andere Funktionsgruppe.

**22.** Eine biologisch abbaubare Beschichtung auf einer Oberfläche eines Medizinproduktes mit: einer biologisch abbaubaren Schicht auf mindestens einem Teilbereich des Medizinproduktes, besteht die Schicht aus einem Copolymer und einem Therapeutikum, welches nach der Implantation des Medizinproduktes in einen Patienten in den Patienten freigesetzt wird, wobei das Copolymer aus einem ersten Monomerbaustein und einem zweiten Monomerbaustein besteht, wobei der erste Monomerbaustein eine Aminosäure und der zweite Monomerbaustein eine derivatisierten Aminosäure aufweist, um eine hydrophobe Kohlenwasserstoffseitenkette mit einem Molekulargewicht von 14 bis 5.000 zu haben.

**23.** Die Beschichtung nach Anspruch 22, wobei das Medizinprodukt nach der Implantation in einen Patienten biologisch abbaubar ist.

**24.** Die Beschichtung nach Anspruch 22, wobei die hydrophobe Kohlenwasserstoffseitenkette eine Alkylkette enthält.

**25.** Die Beschichtung nach Anspruch 22, wobei der erste Monomerbaustein und der zweite Monomerbaustein Bestandteil einer derivatisierten Polyaminosäure sind, um mindesten ein Anteil des Copolymers zu sein.

**26.** Die Beschichtung nach Anspruch 25, wobei die Polyaminosäure ein Homopolymer enthält.

**27.** Die Beschichtung nach Anspruch 25, wobei die Glasübergangstemperatur der Polyaminosäure um mindestens 10 Grad Celsius durch Hinzufügen der hydrophoben Kohlenwasserstoffe zur Polyaminosäure verändert wird.

**28.** Die Beschichtung nach Anspruch 25, wobei die Freigabegeschwindigkeit des Therapeutikums der Polyaminosäure durch Hinzufügen der hydrophoben Kohlenwasserstoffe zur Polyaminosäure verändert wird.

**29.** Die Beschichtung nach Anspruch 25, wobei die hydrophoben Kohlenwasserstoffe in der Polyaminosäure die Löslichkeit der Polyaminosäure in organischen Lösungsmitteln gewähren, wohingegen die Polyaminosäure ohne die hydrophoben Kohlenwasserstoffe in organischen Lösungsmitteln unlöslich ist.

**30.** Die Beschichtung nach Anspruch 22, wobei 3% bis 100% der Seitenketten des Copolymers mit den hydrophoben Kohlenwasserstoffen derivatisiert sind.

**31.** Die Beschichtung nach Anspruch 22, wobei das Copolymer hydrophobe Seitenketten aufweist.

**32.** Die Beschichtung nach Anspruch 31, wobei die hydrophoben Seitenketten Polyethylenoxide, Zuckerreste oder Polysaccharide enthalten.

**33.** Die Beschichtung nach Anspruch 31, wobei die hydrophoben Seitenketten Funktionsgruppen enthalten.

**34.** Die Beschichtung nach Anspruch 33, wobei die Funktionsgruppe aus den Gruppen Hydroxyl, Carboxyl, zwitterionisch, Suphonat, Phosphat und Amino gewählt wird.

**35.** Die Beschichtung nach Anspruch 22, wobei eine chemische Gruppe, die die Aminosäurenseitengruppe und den hydrophoben Kohlenwasserstoff verbindet ein Amid, Ester, Carbonat, Carbamat, Oximester, Acetal, Tetal, Urethan, Urea, Enolester, Oxazolinde oder Anhydrid enthält.

**36.** Die Beschichtung nach Anspruch 22, wobei das Copolymer eine derivatisierte Gamma-Polyaminosäure enthält.

**37.** Die Beschichtung nach Anspruch 36, wobei die Gamma-Polyaminosäure Polyglutaminsäure, Polyasparaginsäure oder Polylysine enthält.

**38.** Die Beschichtung nach Anspruch 22, wobei das Therapeutikum in der Schicht eingeschlossen ist oder kovalent an das Polymer gebunden ist.

**39.** Die Beschichtung nach Anspruch 22, wobei das Copolymer in einem organischen Lösungsmittel löslich und in wässriger Lösung unlöslich ist.

**40.** Die Beschichtung nach Anspruch 22, wobei die erste Aminosäure eine derivatisierte Seitenkette enthält.

**41.** Die Beschichtung nach Anspruch 22, wobei mindestens ein Anteil des Copolymers folgende Formel aufweist:

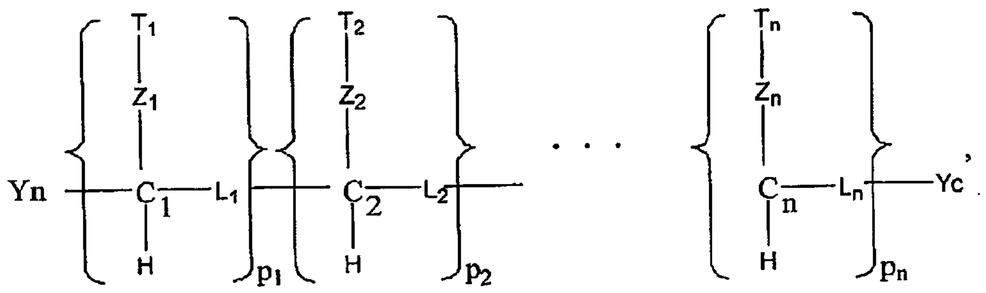

wobei p eine Ganzzahl ist, die einen Monomerbaustein des Polymers anzeigt, und $p_1$ ist der erste Monomerbaustein und $p_2$ ist der zweite Monomerbaustein;
n eine Ganzzahl zwischen 1 und 500.000 ist;
$T_1$... $T_n$ unabhängig ausgewählt sind und eine Formel H oder $-(CH2)_m-X^*$ Gruppe haben,
wobei X* ein H, ein Halogen, eine Hydroxylgruppe, eine Thiolgruppe, eine Carboxylgruppe, eine Aminogruppe, eine Alkylgruppe, eine Alkoxygruppe, eine Alkenylgruppe ist, und $-(CH2)_m-$ ist eine Gruppe, wo m eine Ganzzahl zwischen 1 und 100 einschließlich ist, und eine oder mehrere Methylengruppen wahlweise durch O, S, N, C, C=O, eine $NR_a$ Gruppe, eine $CR_b$ Gruppe, eine $CR_cR_d$ Gruppe oder ein $SiR_eR_f$ ersetzt werden, wobei $R_a$, $R_b$, $R_c$, $R_d$, $R_e$ und $R_f$, jeweils für sich, eine Bindung, eine pu Bindung, H, eine Hydroxylgruppe, eine Thiolgruppe, eine Carboxylgruppe, ein Carbamat, eine Oxocarbon-Gruppe, eine Aminogruppe, eine Amidogruppe, eine Amidgruppe, eine Phosphatgruppe, eine Sulfonatgruppe, eine Alkylgruppe, eine Alkoxygruppe, eine Alkenylgruppe, eine Alkynylgruppe, ein Siloxane oder andere Funktionsgruppe sind;
wobei die hydrophobe Kohlenwasserstoffseitenkette ein Molekulargewicht von 14 bis 5000 hat;
$L_1$ ... $L_n$ sind wahlweise ausgewählt eine Verbindung, oder eine Verbindungsgruppe, die die angezeigten Kohlenstoffe mit kovalenter(n) Bindung(en) verbindet;
$Z_1$... $Z_n$ sind unabhängig ausgewählte Verbindungen oder Verbindungsgruppen mit weniger als 100 Atomen; und $Y_c$ und $Y_n$ sind jeweils unabhängig gewählt H, ein Halogen, eine Hydroxylgruppe, eine Thiolgruppe, eine Carboxylgruppe, eine Aminogruppe, eine Alkylgruppe, eine Alkoxygruppe, eine Alkenylgruppe, eine Alkynylgruppe, ein Siloxane oder andere Funktionsgruppe.

**42.** Ein Medizinprodukt zur Lieferung eines Therapeutikums, hat das Medizinprodukt eine Beschichtung gemäß irgendeines Anspruchs 22, 24 - 41.

**43.** Das Medizinprodukt nach Anspruch 42, wobei die Beschichtung aus einer Schicht des Therapeutikums besteht und eine wasserlösliche Polyaminosäure auf mindestens einem Teilbereich der Oberfläche des Medizinproduktes enthält; die Polyaminosäuren wurden zur Stabilisierung der Schicht miteinander vernetzt, wobei 1% bis 100% der Seitenketten der Polyaminosäuren derivatisiert wurden, um eine hydrophobe Kohlenwasserstoffseitenkette mit einem Molekulargewicht von 14 - 5000 zu haben.

**44.** Das Medizinprodukt nach Anspruch 43, wobei Polyaminosäuren während des Aushärtens der Schicht durch Aufbringung von Wärme- oder Ultraviolettenergie miteinander vernetzt werden.

**45.** Das Medizinprodukt nach Anspruch 42, wobei das Medizinprodukt nach Implantation in einen Patienten biologisch abbaubar ist.

**Revendications**

**1.** Procédé de fabrication d'un revêtement biodégradable sur une surface d'un dispositif médical, comportant :

la formation d'une couche biodégradable sur au moins une partie de la surface du dispositif médical, la couche comprenant un copolymère et un agent thérapeutique libérable dans un patient après implantation du dispositif dans le patient,

où le copolymère comporte une première unité monomère et une deuxième unité monomère, la première unité monomère comportant un acide aminé et la deuxième unité monomère comportant un acide aminé dérivatisé pour avoir une chaîne latérale d'hydrocarbure hydrophobe dotée d'un poids moléculaire allant de 14 à 5000.

2. Procédé de la revendication 1, où le dispositif médical est dégradable après implantation dans un patient.

3. Procédé de la revendication 1, où la chaîne latérale d'hydrocarbure hydrophobe comporte une chaîne alkyle.

4. Procédé de la revendication 1, où on fabrique le copolymère en faisant réagir des chaînes latérales d'un polymère avec des fractions d'hydrocarbures hydrophobes pour former les chaînes latérales d'hydrocarbure hydrophobe du copolymère.

5. Procédé de la revendication 4, où de 3% à 100% des chaînes latérales du polymère sont dérivatisées avec les hydrocarbures hydrophobes.

6. Procédé de la revendication 4, où le polymère est un homopolymère.

7. Procédé de la revendication 1, où le copolymère comporte de plus des chaînes latérales hydrophiles.

8. Procédé de la revendication 7, où les chaînes latérales hydrophiles comportent des polyéthylènes-oxydes, des résidus de sucre ou des polysaccharides.

9. Procédé de la revendication 7, où les chaînes latérales hydrophiles comportent des groupes fonctionnels.

10. Procédé de la revendication 7, où les groupes fonctionnels sont choisis dans le groupe consistant en : hydroxyle, carboxyle, zwittérionique, sulfonate, phosphate et amino.

11. Procédé de la revendication 1, comportant de plus la sélection de la température de transition vitreuse du copolymère en réglant un nombre ou une longueur des chaînes latérales d'hydrocarbure hydrophobe dans le copolymère.

12. Procédé de la revendication 1, comportant de plus la sélection du taux de libération de l'agent thérapeutique de la couche en réglant un nombre ou une longueur des chaînes latérales d'hydrocarbure hydrophobe dans le copolymère.

13. Procédé de la revendication 1, comportant de plus la sélection de la solubilité du copolymère dans un solvant organique en réglant un nombre ou une longueur des chaînes latérales d'hydrocarbure hydrophobe dans le copolymère.

14. Procédé de la revendication 1, où un groupe chimique reliant la chaîne latérale d'acide aminé et l'hydrocarbure hydrophobe comporte un amide, ester, carbonate, carbamate, ester d'oxime, acétal, cétal, uréthane, de l'urée, ester d'énol, des oxazolidines ou un anhydride.

15. Procédé de la revendication 1, où le copolymère comporte un acide gamma-polyaminé dérivatisé.

16. Procédé de la revendication 15, où l'acide gamma-polyaminé comporte de l'acide polyglutamique, de l'acide polyaspartique ou de la polylysine.

17. Procédé de la revendication 1, où l'agent thérapeutique est piégé dans la couche ou lié par covalence au copolymère.

18. Procédé de la revendication 1, où le copolymère est soluble dans un solvant organique et insoluble dans une solution aqueuse.

19. Procédé de la revendication 1, où l'acide aminé de la première unité monomère comprend une chaîne latérale dérivatisée.

**20.** Procédé de la revendication 1, où de 5% à 95% de tous les acides aminés du copolymère comprennent une chaîne latérale dérivatisée.

**21.** Procédé de la revendication 1, où au moins une partie du copolymère comporte une formule de :

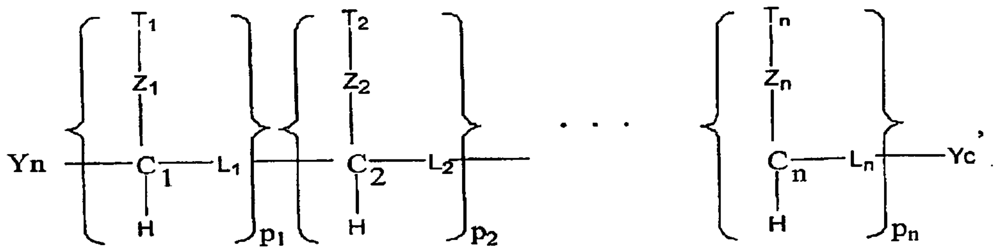

où :

p est un nombre entier qui indique une unité monomère du polymère, et $p_1$ indique la première unité monomère et $p_2$ indique la deuxième unité monomère ;
n est un nombre entier entre 1 et 500 000 ;
$T_1$... $T_n$ sont choisis indépendamment pour avoir une formule de H ou groupe -$(CH2)_m$-X*,
où X* est H, un halogène, un groupe hydroxyle, un groupe thiol, un groupe carboxyle, un groupe aminé, un groupe alkyle, un groupe alcoxyle, un groupe alcényle, un groupe alcynyle, et -$(CH2)_m$- est un groupe où m est un nombre entier allant de 1 à 100 compris, et un ou plusieurs des groupes méthylène est remplacé en option par 0, S, N, C, C=O, un groupe $NR_a$, un groupe $CR_b$, un groupe $CR_cR_d$, ou un $SiR_eR_f$ où $R_a$, $R_b$, $R_c$, $R_d$, $R_e$, et $R_f$ sont, chacun indépendamment, un liant, un liant de pi, H, un groupe hydroxyle, un groupe thiol, un groupe carboxyle, un carbamate, un groupe oxocarbone, un groupe aminé, un groupe amido, un groupe amide, un groupe phosphate, un groupe sulfonate, un groupe alkyle, un groupe alcoxyle, un groupe alcényle, un groupe alcynyle, un siloxane ou un autre groupe fonctionnel ;
où la chaîne latérale d'hydrocarbure hydrophobe a un poids moléculaire allant de 14 à 5000 ;
$L_1$ ... $L_n$ sont choisis indépendamment pour être un liant, tel qu'un groupe de liaison qui lie les carbones indiqués par une ou des liaisons covalentes ;
$Z_1$... $Z_n$ sont choisis indépendamment pour être un liant ou un groupe de liaison de moins de 100 atomes ; et
$Y_c$ et $Y_n$ sont chacun choisis indépendamment pour être H, un halogène, un groupe hydroxyle, un groupe thiol, un groupe carboxyle, un groupe aminé, un groupe alkyle, un groupe alcoxyle, un groupe alcényle, un groupe alcynyle, un siloxane ou un autre groupe fonctionnel.

**22.** Revêtement biodégradable sur une surface d'un dispositif médical, comprenant : une couche biodégradable sur au moins une partie de la surface du dispositif médical, la couche comprenant un copolymère et un agent thérapeutique libérable dans un patient après implantation du dispositif dans le patient,
où le copolymère comporte une première unité monomère et une deuxième unité monomère, la première unité monomère comportant un acide aminé et la deuxième unité monomère comportant un acide aminé dérivatisé pour comporter une chaîne latérale d'hydrocarbure hydrophobe dotée d'un poids moléculaire allant de 14 à 5000.

**23.** Revêtement de la revendication 22, où le dispositif médical est dégradable après implantation dans un patient.

**24.** Revêtement de la revendication 22, où la chaîne latérale d'hydrocarbure hydrophobe comporte une chaîne alkyle.

**25.** Revêtement de la revendication 22, où la première unité monomère et la deuxième unité monomère font partie d'un acide polyaminé dérivatisé pour former au moins une partie du copolymère.

**26.** Revêtement de la revendication 25, où l'acide polyaminé comprend un homopolymère.

**27.** Revêtement de la revendication 25, où la température de transition vitreuse de l'acide polyaminé est modifiée d'au moins 10 degrés Centigrade par l'ajout des hydrocarbures hydrophobes à l'acide polyaminé.

**28.** Revêtement de la revendication 25, où le taux de libération de l'agent thérapeutique de l'acide polyaminé est modifié

par l'ajout des hydrocarbures hydrophobes à l'acide polyaminé.

**29.** Revêtement de la revendication 25, où les hydrocarbures hydrophobes sur l'acide polyaminé confèrent de la solubilité dans des solvants organiques à l'acide polyaminé, où l'acide polyaminé est insoluble dans le solvant organique sans les hydrocarbures hydrophobes.

**30.** Revêtement de la revendication 22, où de 3% à 100% des chaînes latérales du copolymère sont dérivatisées avec les hydrocarbures hydrophobes.

**31.** Revêtement de la revendication 22, où le copolymère comprend de plus des chaînes latérales hydrophiles.

**32.** Revêtement de la revendication 31, où les chaînes latérales hydrophiles comportent des polyéthylènes-oxydes, des résidus de sucre ou des polysaccharides.

**33.** Revêtement de la revendication 31, où les chaînes latérales hydrophiles comportent des groupes fonctionnels.

**34.** Revêtement de la revendication 33, où les groupes fonctionnels sont choisis dans le groupe consistant en : hydroxyle, carboxyle, zwittérionique, sulfonate, phosphate et amino.

**35.** Revêtement de la revendication 22, où un groupe chimique reliant la chaîne latérale d'acide aminé et l'hydrocarbure hydrophobe comporte un amide, ester, carbonate, carbamate, ester d'oxime, acétal, cétal, uréthane, de l'urée, ester d'énol, des oxazolidines ou un anhydride.

**36.** Revêtement de la revendication 22, où le copolymère comporte un acide gamma-polyaminé dérivatisé.

**37.** Revêtement de la revendication 36, où l'acide gamma-polyaminé comporte de l'acide polyglutamique, de l'acide polyaspartique ou de la polylysine.

**38.** Revêtement de la revendication 22, où l'agent thérapeutique est piégé dans la couche ou lié par covalence au copolymère.

**39.** Revêtement de la revendication 22, où le copolymère est soluble dans un solvant organique et insoluble dans une solution aqueuse.

**40.** Revêtement de la revendication 22, où le premier acide aminé comprend une chaîne latérale dérivatisée.

**41.** Revêtement de la revendication 22, où au moins une partie du copolymère comporte une formule de :

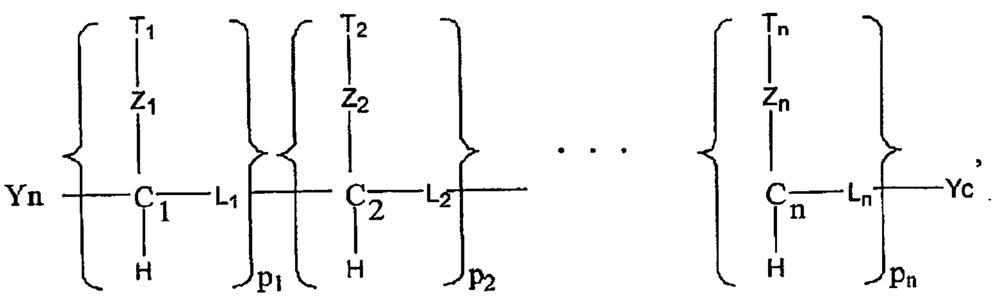

où p est un nombre entier qui indique une unité monomère du polymère, et $p_1$ indique la première unité monomère et $p_2$ indique la deuxième unité monomère ;
n est un nombre entier entre 1 et 500 000 ;
$T_1$... $T_n$ sont choisis indépendamment pour avoir une formule de H ou groupe $-(CH2)_m-X^*$,
où X* est H, un halogène, un groupe hydroxyle, un groupe thiol, un groupe carboxyle, un groupe aminé, un groupe alkyle, un groupe alcoxyle, un groupe alcényle, un groupe alcynyle, et $-(CH2)_m-$ est un groupe où m est un nombre entier allant de 1 à 100 compris, et un ou plusieurs des groupes méthylène est remplacé en option par 0, S, N, C, C=O, un groupe $NR_a$, un groupe $CR_b$, un groupe $CR_cR_d$, ou un $SiR_eR_f$ où $R_a$, $R_b$, $R_c$, $R_d$, $R_e$, et $R_f$ sont, chacun

indépendamment, un liant, un liant de pi, H, un groupe hydroxyle, un groupe thiol, un groupe carboxyle, un carbamate, un groupe oxocarbone, un groupe aminé, un groupe amido, un groupe amide, un groupe phosphate, un groupe sulfonate, un groupe alkyle, un groupe alcoxyle, un groupe alcényle, un groupe alcynyle, un siloxane ou un autre groupe fonctionnel;
où la chaîne latérale d'hydrocarbure hydrophobe a un poids moléculaire allant de 14 à 5000 ;
$L_1$ ... $L_n$ sont choisis indépendamment pour être un liant, ou un groupe de liaison qui lie les carbones indiqués par une ou des liaisons covalentes ;
$Z_1$... $Z_n$ sont choisis indépendamment pour être un liant ou un groupe de liaison de moins de 100 atomes ;
et $Y_c$ et $Y_n$ sont chacun choisis indépendamment pour être H, un halogène, un groupe hydroxyle, un groupe thiol, un groupe carboxyle, un groupe aminé, un groupe alkyle, un groupe alcoxyle, un groupe alcényle, un groupe alcynyle, un siloxane ou un autre groupe fonctionnel.

**42.** Dispositif médical conçu pour distribuer un agent thérapeutique, le dispositif médical comprenant un revêtement conforme à une quelconque des revendications 22, 24 à 41.

**43.** Dispositif médical de la revendication 42 où le revêtement comprend une couche de l'agent thérapeutique et des acides polyaminés solubles dans l'eau sur au moins une partie d'une surface du dispositif médical; les acides polyaminés ayant été réticulés l'un à l'autre afin de stabiliser la couche, où entre 1% et 100% des chaînes latérales des acides polyaminés sont dérivatisés pour avoir une chaîne latérale d'hydrocarbure hydrophobe dotée d'un poids moléculaire allant de 14 à 5000.

**44.** Dispositif médical de la revendication 43 où des acides polyaminés ont été réticulés l'un à l'autre à la cuisson de la couche avec une application de chaleur ou d'énergie ultraviolette.

**45.** Dispositif médical de la revendication 42 où le dispositif médical est dégradable après implantation dans un patient.

Figure 1

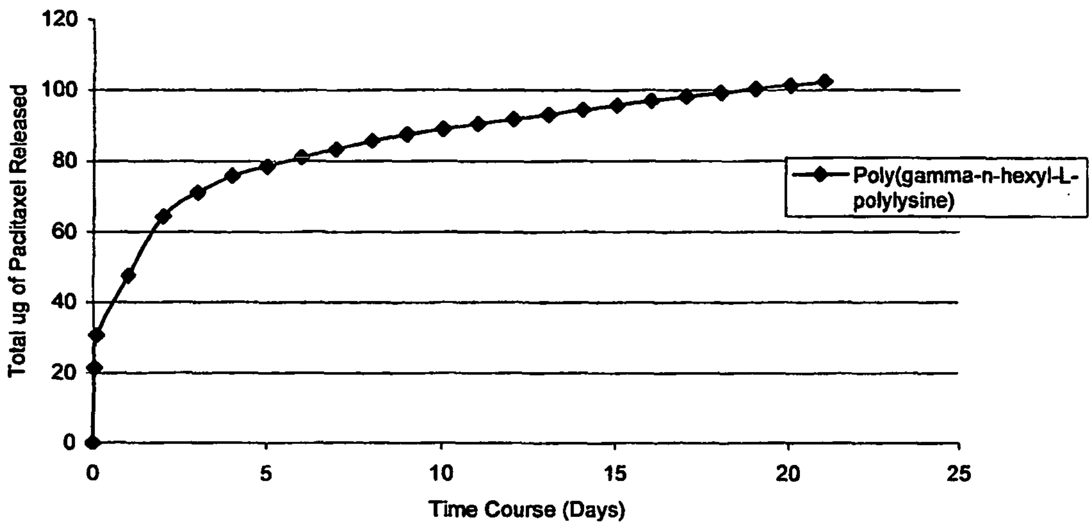


Figure 2

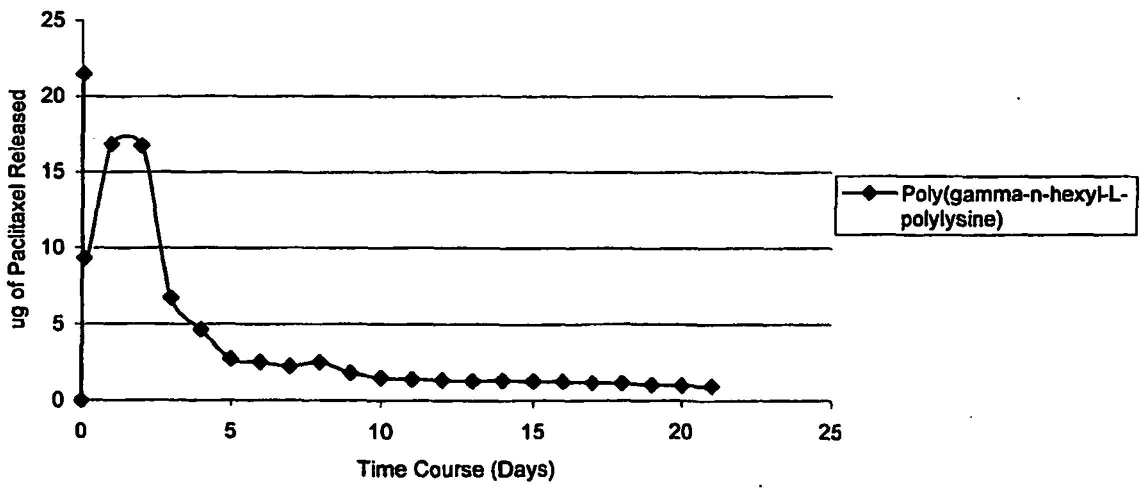

Figure 3

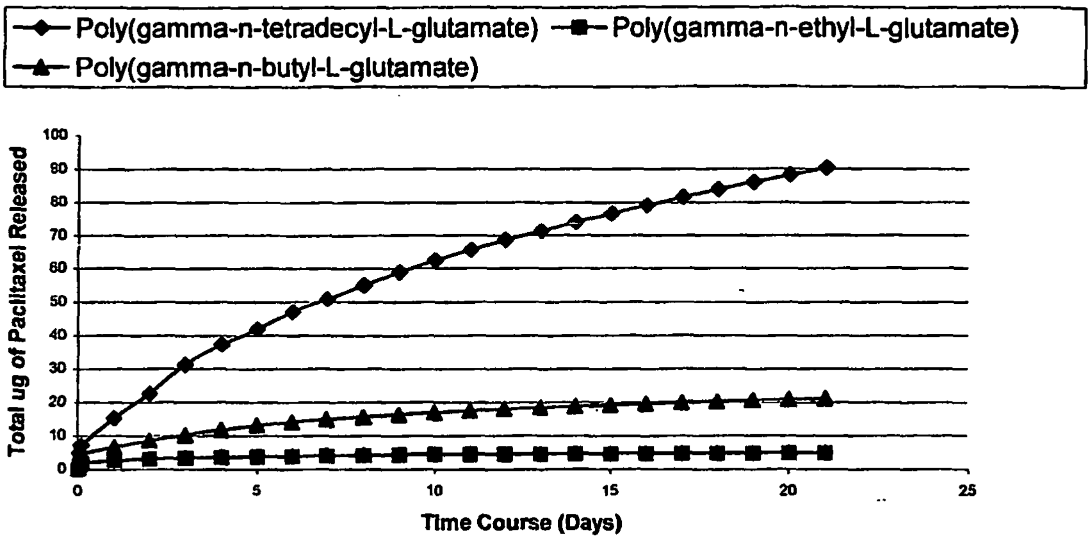


Figure 4

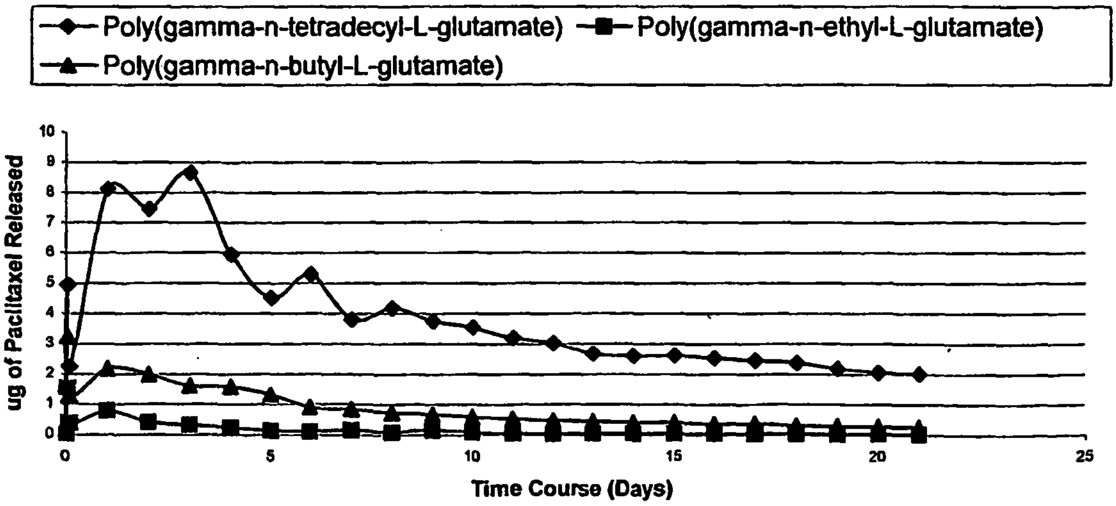

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03042277 A
- WO 03072156 A
- US 4888398 A
- WO 9847948 A
- JP 1170464 A
- US 5464650 A
- US 5900246 A
- US 6214901 B
- US 6517858 B
- US 20020002353 A
- WO 0187342 A2
- WO 03024500 A
- US 60574250 B
- US 5782908 A
- US 5980972 A
- US 6231600 B
- US 6251136 B
- US 6387379 B
- US 6503556 B
- US 3285953 A, D. Wasserman, Springfield, J.D. Garber, F.M. Meigs


### Non-patent literature cited in the description


- **Tosatti et al.** *Biomaterials,* 2003, vol. 27, 4949-4958
- **Yashura et al.** *Chemistry Express,* 1992, vol. 7, 753-756
- **M. Bodanszky.** Peptide Chemistry. Springer-Verlag, 1988
- **Norbert Sewald.** Peptides: Chemistry and Biology. Wiley, John & Sons, August 2002
- **Gregory A. Grant.** Synthetic Peptides: A User's Guide. Oxford University Press, March 2002
- **T. W. Greene.** Protective Groups in Organic Synthesis. Wiley, 1999
- **R. C. Larock.** Comprehensive Organic Transformations: A Guide to Functional Group Preparations. 1989
- Comprehensive Organic Synthesis: Selectivity, Strategy & Efficiency in Modem Organic Chemistry: Cumulative Indexes. vol. 9
- **Y. Wolman.** *Journal of Lipid Research,* 1967, vol. 3, 142-145
- **D. Derrieu ; P. Midoux ; C. Petit ; E. Negre ; R. Mayer ; M. Monsigny ; A-C Roche.** *Glycoconjugate Journal,* 1989, vol. 6, 241-255
- **S. Snyder ; P. Sobocinski.** *Anal. Biochem,* 1975, vol. 64, 284-288
- **W.D. Fuller ; M.S. Verlander ; M. Goodman.** *Biopolymers,* 1976, vol. 15, 1869-1871
- **T. Shimokuri ; T. Kaneko ; T. Serizawa ; M. Akashi.** *Macromolecular Bioscience,* 2004, vol. 4, 407-4116
- **T. Shimokuri ; T. Kaneko ; T. Serizawa ; M. Akashi.** *Macromolecular Bioscience,* 2004, vol. 4, 407-411